# EUROPEAN PATENT SPECIFICATION

(11) **EP 2 904 092 B1**
(45) Date of publication and mention of the grant of the patent: **20.09.2017**
(21) Application number: 13776656.4
(22) Date of filing: 27.09.2013
(51) Int. Cl.: C07K 16/28, C07K 16/00

(54) **COMPOSTIONS AND METHODS FOR PRODUCING GLYCOPROTEINS**
ZUSAMMENSETZUNGEN UND VERFAHREN ZUR HERSTELLUNG VON GLYCOPROTEINEN
COMPOSITIONS ET PROCÉDÉS DE PRODUCTION DE GLYCOPROTÉINES

(30) Priority: 01.10.2012 US 201261708554 P
(43) Date of publication of application: 12.08.2015
(60) Divisional application: 17184980.5
(73) Proprietor: AbbVie Biotherapeutics Inc., Redwood City, CA 94063 (US)
(72) Inventor: VARMA, Amit, Fremont, California 94536 (US); CUENCA, James, Union City, California 94587 (US); ZHU, Ying, Palo Alto, California 94301 (US)
(74) Representative: J A Kemp
(86) International application number: PCT/US2013/062410
(87) International publication number: WO 2014/055370

(56) References cited:
- WO-A2-02/066603
- SHIELDS ROBERT L ET AL: "Lack of fucose on human IgG1 N-linked oligosaccharide improves binding to human Fcgamma RIII and antibody-dependent cellular toxicity", JOURNAL OF BIOLOGICAL CHEMISTRY, AMERICAN SOCIETY FOR BIOCHEMISTRY AND MOLECULAR BIOLOGY, US, vol. 277, no. 30, 26 July 2002 (2002-07-26), pages 26733-26740, XP002442140, ISSN: 0021-9258, DOI: 10.1074/JBC.M202069200
- GASDASKA JOHN R ET AL: "An afucosylated anti-CD20 monoclonal antibody with greater antibody-dependent cellular cytotoxicity and B-cell depletion and lower complement-dependent cytotoxicity than rituximab.", MOLECULAR IMMUNOLOGY MAR 2012, vol. 50, no. 3, March 2012 (2012-03), pages 134-141, XP002716094, ISSN: 1872-9142
- JINYOU ZHANG ET AL: "Development of Animal-free, Protein-Free and Chemically-Defined Media for NS0 Cell Culture", CYTOTECHNOLOGY, KLUWER ACADEMIC PUBLISHERS, DO, vol. 48, no. 1-3, 1 June 2005 (2005-06-01) , pages 59-74, XP019236890, ISSN: 1573-0778, DOI: 10.1007/S10616-005-3563-Z
- WUU JESSICA J ET AL: "Altering mAb glycosylation through cell culture media additives", ABSTRACTS OF PAPERS AMERICAN CHEMICAL SOCIETY, vol. 239, March 2010 (2010-03), pages 357-BIOT, XP009174078, & 239TH NATIONAL MEETING OF THE AMERICAN-CHEMICAL-SOCIETY; SAN FRANCISCO, CA, USA; MARCH 21 -25, 2010 ISSN: 0065-7727
- SERRATO J ANTONIO ET AL: "Differences in the glycosylation profile of a monoclonal antibody produced by hybridomas cultured in serum-supplemented, serum-free or chemically defined media.", BIOTECHNOLOGY AND APPLIED BIOCHEMISTRY JUN 2007, vol. 47, no. Pt 2, June 2007 (2007-06), pages 113-124, XP002716095, ISSN: 1470-8744
- James Cuenca: "Method for Improved Control of Anti-Tumor Effector Function of MAb By Modification of NS0 Cell Culture Medium", Meeting Abstract (13AIChE Annual meeting) , November 2013 (2013-11), XP002716096, Retrieved from the Internet: URL:https://aiche.confex.com/aiche/2013/we bprogram/Paper316355.html [retrieved on 2013-11-08]

## Description

### 2. BACKGROUND

Post-translational modification of proteins through glycosylation leads to the formation of distinct glycoforms, which often have unique properties. Differences in glycosylation of proteins can have a profound influence on the physical and chemical properties of the protein, including the binding affinity of the protein to a particular target molecule. Several methods of modulating glycosylation of proteins are known. Different expression systems can impart distinct glycosylation profiles. Moreover, protein coding sequences can be modified to produce proteins with an enhanced or decreased level of a particular glycoform. The conditions under which recombinant cell lines are cultured can also influence the glycosylation profile.

Antibodies produced recombinantly in mammalian cells are often glycosylated at both the Fc and the Fab regions of the antibody. Many glycosylated antibodies contain the sugar fucose. The presence of fucose in an antibody can influence the binding of the antibody to particular proteins on cells. For instance, antibodies containing high levels of fucose in the Fc region of the antibody can have diminished binding to receptors on lymphocytes (*e.g*., natural killer cells). Antibodies lacking fucose have been correlated with enhanced ADCC (antibody-dependent cellular cytotoxicity) activity, especially at low doses of antibody. Shields et al., 2002, J. Biol. Chem. 277:26733-26740; Shinkawa et al., 2003, J. Biol. Chem. 278:3466. Methods of preparing fucose-less antibodies include growth in rat myeloma YB2/0 cells (ATCC CRL 1662). YB2/0 cells express low levels of FUT8 mRNA, which encodes an enzyme (α 1,6-fucosyltransferase) necessary for fucosylation of proteins. Alternative methods for increasing ADDC activity include mutations in the Fc portion of an antibody, particularly mutations which increase antibody affinity for an FcγR receptor. A correlation between increased FcγR binding with mutated Fc has been demonstrated using targeted cytoxicity cell-based assays. Shields et al., 2001, J. Biol. Chem. 276:6591-6604; Presta et al., 2002, Biochem Soc. Trans. 30:487-490.

The concentration of components in a culture medium or feed medium may influence the glycosylation profile of a protein. Ideally, a particular glycoform of a protein may be enhanced or diminished through control of the conditions under which recombinant cell lines are grown. While there have been a multitude of studies directed to increasing the yields of proteins through manipulating the components of a culture medium, there have been far fewer reports concerned with modulating glycosylation of proteins through modifying the cell culture medium in which recombinantly engineered cells are cultured. One example is disclosed in U.S. Patent No. 5,705,364, which is directed to a process for controlling the content of sialic acid of glycoproteins produced by mammalian cells through the addition of an alkanoic acid to the culture medium. However, the possibility of increasing ADCC activity of antibodies through modifications of the culture media in which recombinant antibodies are expressed has not been sufficiently addressed. Accordingly, developing a cell culture medium for culturing mammalian cells to express antibodies with reduced levels of fucose and increased ADCC activity would be highly desirable.
WO 02/066603 discloses methods and compositions for chemically defined media for growth of mammalian cells for production of commercially useful amounts of expressed proteins.

### 3. SUMMARY OF THE DISCLOSURE

The present disclosure relates to the discovery that glycoproteins (e.g., antibodies) with beneficial properties are produced by culturing mammalian cells in a culture medium comprising a high concentration (*e.g*., at least 5 mM) of glycine, (hereinafter "high glycine medium"). In particular, culturing mammalian host cells in high glycine media increases levels of non-fucosylated glycoforms. The mammalian cells can be used to produce antibodies that display enhanced biological activity, including enhanced antibody dependent cellular cytotoxicity ("ADCC") activity.

Accordingly, the disclosure generally provides methods and compositions for expressing recombinant proteins (*e.g.,* antibodies) with modified glycosylation profiles as compared to glycoproteins expressed in traditional culture media. The methods and compositions utilize high glycine media to express glycoproteins, such as antibodies, in mammalian cells, advantageously result in glycoproteins with reduced fucosylation levels as compared to glycoproteins produced in traditional culture media.
Thus, in accordance with the present invention, there is provided a method of producing an IgG1 antibody, comprising culturing NSO cells engineered to secrete and express the IgG1 antibody in a cell culture medium comprising glycine at a concentration between 5 mM and 30 mM under conditions suitable for expression and secretion of the IgG1 antibody, thereby producing the IgG1 antibody.

Additionally, the methods of the disclosure can be used to modulate and control protein glycosylation during manufacturing. Improved control of product glycosylation during manufacturing is desirable since it will reduce the risk of batch rejection and improve control during technology transfer between manufacturing sites and during scale-up of the process. Therefore, the methods can be used to maintain reproducible product glycosylation profiles without introducing process changes.

Mammalian cells capable of being engineered to produce recombinant glycoproteins include NS0 cells, CHO cells, mouse myeloma SP2/0 cells, baby hamster kidney BHK-21 cells and human embryonic kidney HEK0291 cells. In particular embodiments, NS0 cells are recombinantly engineered to produce antibodies.

In one aspect, the disclosure provides culture media suitable for mammalian cell culture comprising glycine at a concentration between 5 mM and 100 mM and optionally other amino acids at various concentrations as indicated in the disclosure. The culture medium is preferably a chemically-defined, protein free medium. The concentration of glycine in the cell culture medium can be, for instance, 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 22 mM, 25 mM, 30 mM or 50 mM. In certain embodiments, the concentration of glycine in the cell culture medium is bounded by any of the two foregoing embodiments, *e.g.,* a concentration ranging from 5 mM to 10 mM, from 7 mM to 10 mM, from 10 mM to 15 mM, from 12 mM to 17 mM, from 15 mM to 20 mM, from 16 mM to 18 mM from 10 mM to 30 mM, from 10 mM to 25 mM, from 20 mM to 30 mM, from 10 mM to 50 mM, from 20 mM to 50 mM, *etc.* Other amino acids can be present in the culture medium, generally at concentrations typically found in a traditional basal medium. In one embodiment, concentrations of the amino acids in the culture media of the present disclosure are found in ranges listed in Table 2. The culture medium can also include one or more additional components such as vitamins, trace elements, energy sources, fatty acids, growth factors, nucleosides, lipids, hormones and/or antibiotics. Furthermore, the medium can include a buffer, an osmolality regulator and/or a surfactant. Further details of such additional components can be found in Section 5.1.

In another aspect, one or more amino acids other than glycine have concentrations at or below the concentrations shown in Table 3 or Table 4. For instance, two, three, four, five, six, seven, eight, nine, ten or eleven amino acids can have concentrations at or below the concentrations listed in Table 3 or Table 4. In one embodiment, the concentration of lysine in the culture medium is between 0.5 mM and 5.0 mM.

In another aspect, the present disclosure provides methods for producing a glycoprotein of interest ("GOI"), *e.g.* an antibody of interest ("AOI"), comprising culturing mammalian cells, *e.g.* NS0 cells, engineered to secrete and express the GOI or AOI in a cell culture medium comprising glycine at a concentration of at least 5 mM, for example 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 22 mM, 25 mM or 30 mM. The AOI can be, for instance, a murine antibody, a humanized antibody, a chimeric antibody or a fully human antibody. In specific embodiments, the concentration of glycine in the cell culture medium is bounded by any of the two foregoing embodiments, *e*.*g*., a concentration of glycine ranging from 5 mM to 10 mM, from 7 mM to 10 mM, from 10 mM to 15 mM, from 12 mM to 17 mM, from 15 mM to 20 mM, from 16 mM to 18 mM, from 10 mM to 30 mM, from 10 mM to 25 mM, from 20 mM to 30 mM, *etc.* In certain embodiments, the culture medium is a chemically defined protein free medium.

In one embodiment, the AOI is HuLuc63 (elotuzumab). The amino acid sequence of the mature V_{H} chain of elotuzumab (SEQ ID NO:1), the mature V_{L} chain of elotuzumab (SEQ ID NO:2), the full heavy chain sequence of elotuzumab (SEQ ID NO:3) and the full light chain sequence of elotuzumab (SEQ ID NO:4) are shown in Table 7. The cell culturing processes of the present disclosure produce a population of elotuzumab molecules in which at least 4% of the glycans lack fucose and in which at least 2.5% of the glycans are Mannose5 glycans. In one embodiment, the cell culturing processes of the present disclosure produce a population of elotuzumab molecules in which at least 5% of the glycans lack fucose and in which at least 3% of the glycans are Mannose5 glycans.

In another embodiment, the antibody of interest is daclizumab. The amino acid sequence of the mature V_{H} chain of daclizumab (SEQ ID NO:5), the mature V_{L} chain of daclizumab (SEQ ID NO:6), the full heavy chain sequence of daclizumab (SEQ ID NO:7) and the full light chain sequence of daclizumab (SEQ ID NO:8) are shown in Table 7. The cell culturing process of the present disclosure produces a population of daclizumab molecules in which at least 3% of the glycans lack fucose and in which at least 1.5% of the glycans are Mannose5 glycoforms. In one embodiment, the cell culturing process of the present disclosure produces a population of daclizumab molecules in which at least 4% of the glycans lack fucose and in which at least 2% of the glycans are Mannose5 glycans.

In another embodiment, the AOI is voloxicimab. The amino acid sequence of the mature V_{H} chain of voloxicimab (SEQ ID NO:9), the mature V_{L} chain of voloxicimab (SEQ ID NO:10), the full heavy chain sequence of voloxicimab (SEQ ID NO:11) and the full light chain sequence of voloxicimab (SEQ ID NO:12) are shown in Table 7. The cell culturing processes of the present disclosure produce a population of voloxicimab molecules in which at least 1% of the molecules lack fucose and in which at least 0.5% of the glycans are Mannose5 glycans.

In a further embodiment, the AOI is PDL241. The amino acid sequence of the mature V_{H} chain of PDL241 (SEQ ID NO:13), the mature V_{L} chain of PDL241 (SEQ ID NO:14), the full heavy chain sequence of PDL241 (SEQ ID NO:15), the full light chain sequence of PDL241 (SEQ ID NO:16) are shown in Table 7. The cell culturing processes of the present disclosure produce a population of PDL241 molecules in which at least 14% of the glycans lack fucose and in which at least 8% of the glycans are Mannose5 glycans. In one embodiment, the cell culturing processes of the present disclosure produce a population of PDL241 molecules in which at least 15% of the glycans lack fucose and in which at least 9% of the glycans are Mannose5 glycans.

In yet another embodiment, the AOI is PDL192 (enavatuzumab). The amino acid sequence of the mature V_{H} chain of enavatuzumab (SEQ ID NO:17), the mature V_{L} chain of enavatuzumab (SEQ ID NO:18), the full heavy chain sequence of enavatuzumab (SEQ ID NO:19) and the full light chain sequence of enavatuzumab (SEQ ID NO:20) are shown in Table 7. The cell culturing processes of the present disclosure produce a population of enavatuzumab molecules in which at least 5% of the glycans lack fucose and in which at least 2.5% of the glycans are Mannose5 glycans. In one embodiment, the cell culturing processes of the present disclosure produce a population of enavatuzumab molecules in which at least 6% of the glycans lack fucose and in which at least 3% of the glycans are Mannose5 glycans.

### 4. BRIEF DESCRIPTION OF FIGURES

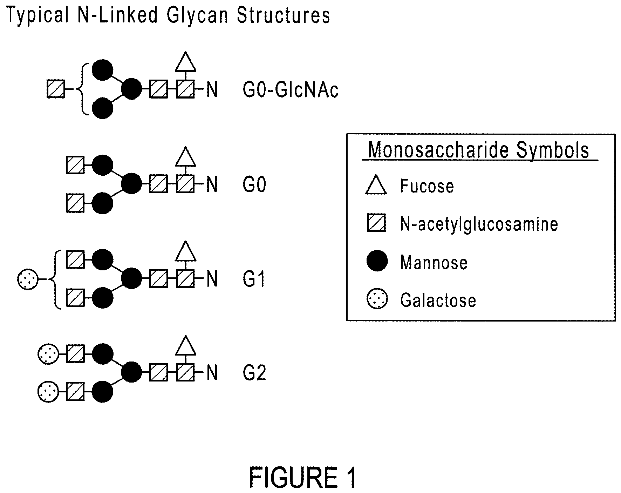

**FIGURE 1** shows the structure of some typical N-linked glycans.

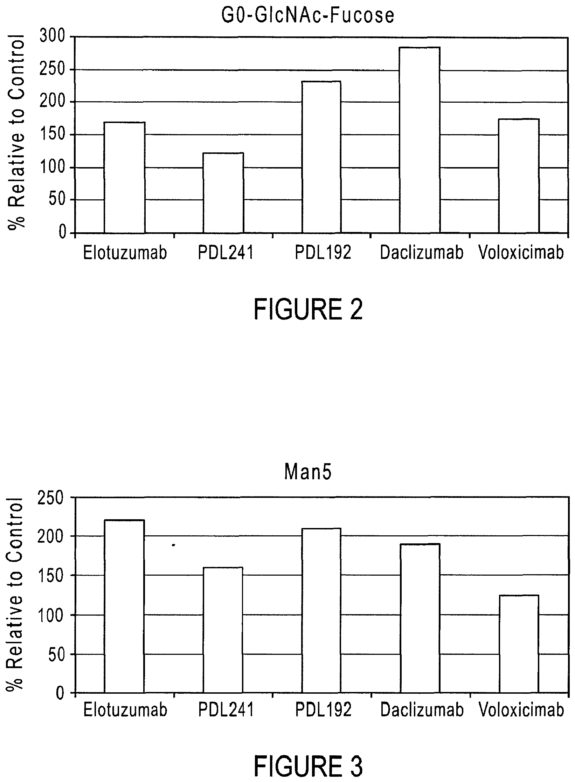

**FIGURE 2** shows the relative amount of non-fucosylated G0 glycans without GlcNAc (N-acetylglucosamine) attached in five different antibodies produced by NS0 cells cultured in basal medium supplemented with additional glycine. The amount is expressed as a percentage relative to the amount of the glycans present in antibodies produced by NS0 cells cultured in control basal medium without additional glycine supplementation.
**FIGURE 3** shows the relative amount of Mannose5 (Man5) glycans attached in five different antibodies produced by NS0 cells cultured in basal medium with a glycine concentration of 17mM. The amount is expressed as a percentage relative to the amount of the glycans present in antibodies produced by NS0 cells cultured in control basal medium without additional glycine supplementation.

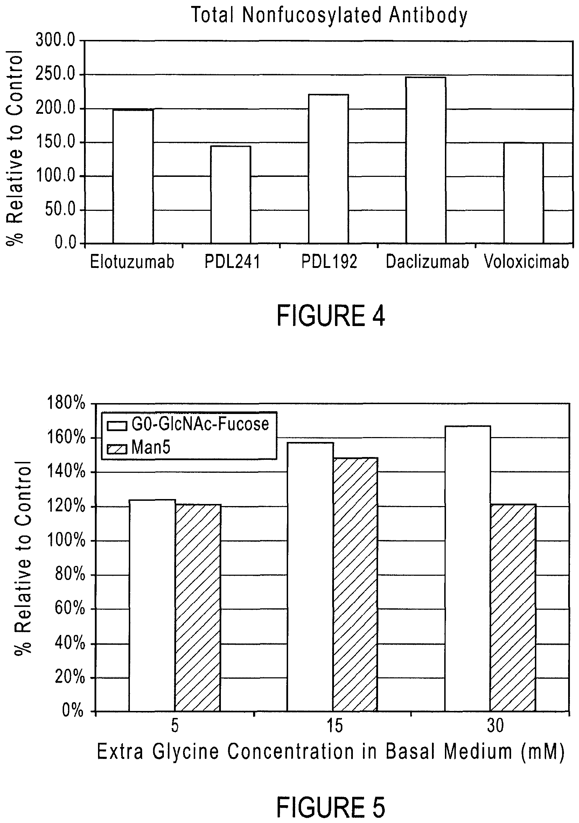

**FIGURE 4** shows the relative amount of total non-fucosylated glycans attached in five different antibodies produced by NS0 cells cultured in basal medium s with a glycine concentration of 17 mM. The amount is expressed as a percentage relative to the amount of the glycans present in antibodies produced by NS0 cells cultured in control basal medium without additional glycine supplementation.
**FIGURE 5** shows the relative amounts of non-fucosylated G0 glycoform without GlcNAc (N-acetylglucosamine) and Man5-type glycoforms attached in PDL192 produced by NS0 cells cultured in basal medium supplemented with different concentrations of glycine (5, 15, 30 mM). The amount is expressed as a percentage relative to the amount of the glycoform present in antibodies produced by NS0 cells cultured in control basal medium without additional glycine supplementation.

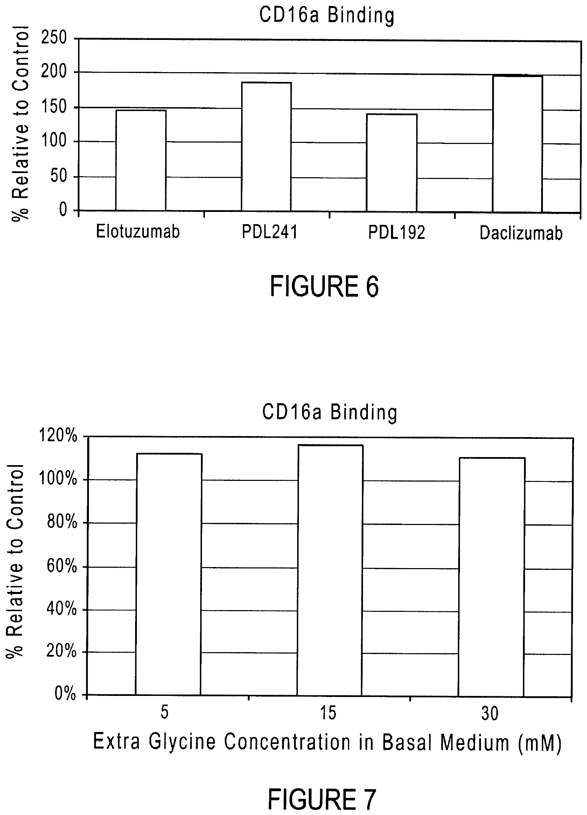

**FIGURE 6** shows the relative binding potency to the CD16a receptor for four IgG1 antibodies (elotuzumab, daclizumab, PDL192 and PDL241) produced by NS0 cells cultured in basal medium supplemented with additional glycine. The amount is expressed as a percentage relative to the CD16a binding affinities of the same antibodies produced by NS0 cells cultured in control basal medium without additional glycine supplementation.
**FIGURE 7** shows the relative binding potency to the CD16a receptor for PDL192 (enavatuzumab) produced by NS0 cells cultured in basal medium supplemented with different concentrations of glycine (5, 15, 30 mM). The amount is expressed as a percentage relative to the CD16a binding affinities of the same antibodies produced by NS0 cells cultured in control basal medium without additional glycine supplementation.

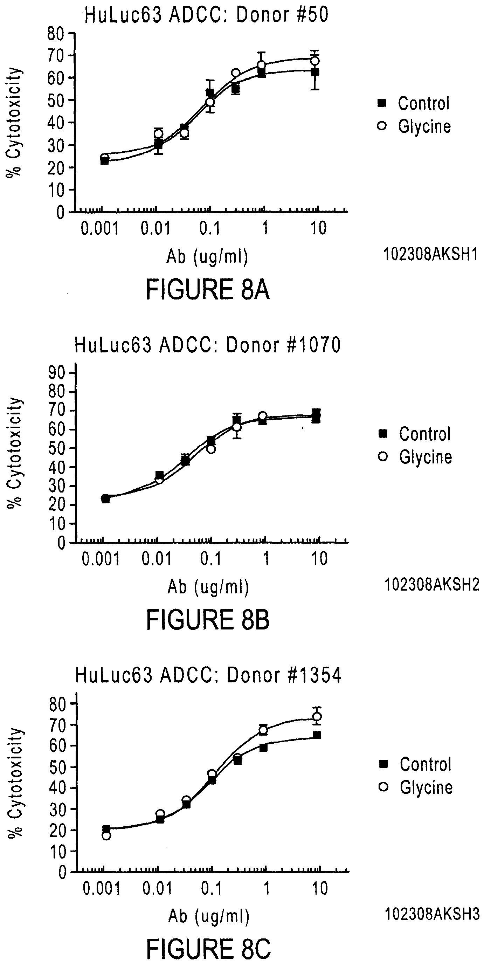

**FIGURES 8A-C** show the ADCC activity of elotuzumab produced from both the control and high glycine process. The studies were performed on human peripheral blood mononuclear cells (PBMCs) from three different donors. Negative controls were included in the assays using a control antibody without ADCC activity and samples without antibody addition.

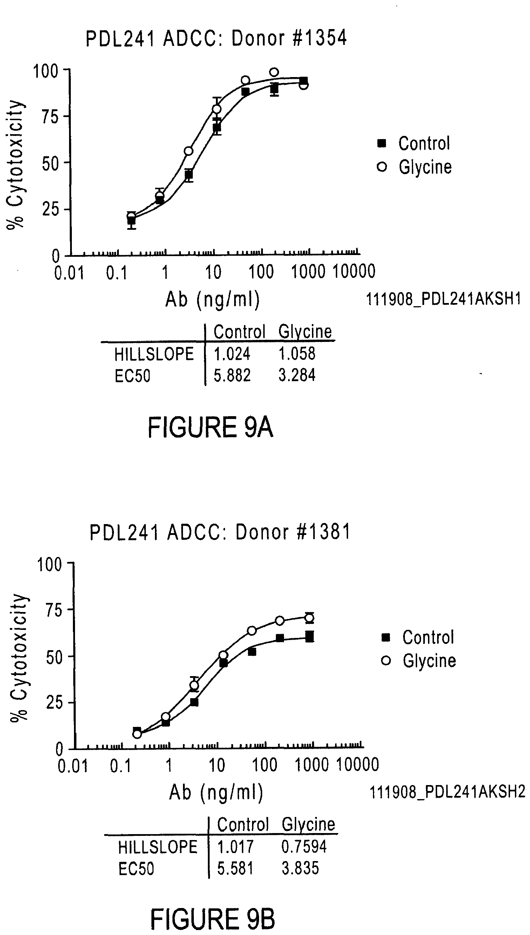

**FIGURES 9A-B** show the ADCC activity of PDL 241 produced from both the control and high glycine process. The studies were performed on PBMC from two different donors. Negative controls were included in the assays using a control antibody without ADCC activity and samples without antibody addition.

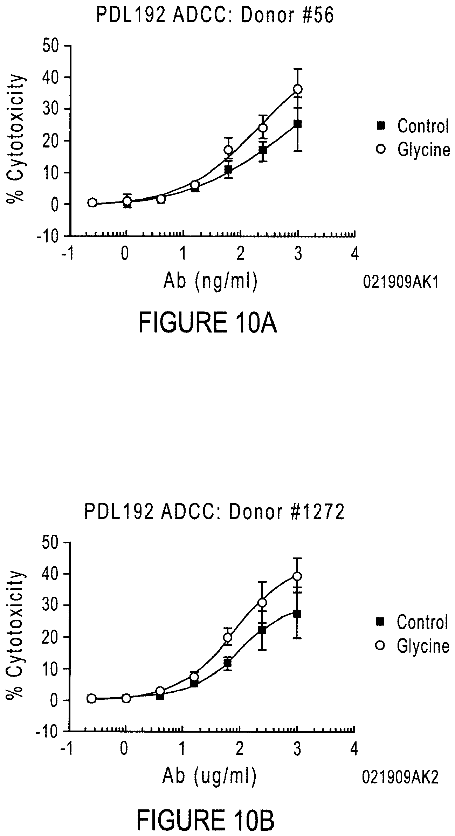

**FIGURES 10A-B** show the ADCC activity of PDL192 (enavatuzumab) produced from both the control and high glycine process. The studies were performed on PBMC from two different donors. Negative controls were included in the assays using a control antibody without ADCC activity and samples without antibody addition.

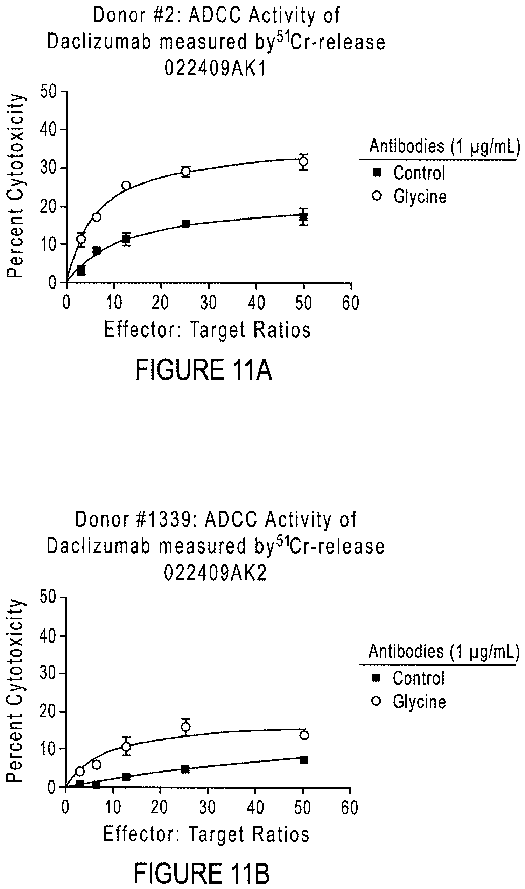

**FIGURES 11A-B** show the ADCC activity of daclizumab produced from both the control and high glycine process. The studies were performed on PBMC from two different donors. Negative controls were included in the assays using a control antibody without ADCC activity and samples without antibody addition.

### 5. DETAILED DESCRIPTION OF THE DISCLOSURE

The present disclosure relates to compositions and methods for recombinant glycoprotein (e.g., antibody) expression. The compositions and methods produce glycoproteins with reduced fucosylated glycoforms and increased ADCC activity as compared to glycoprotein produced in traditional culture media. Antibodies produced by expression systems utilizing the compositions and methods of the disclosure advantageously display less fucosylation than when produced by culturing in traditional media. Various aspects of the disclosure are described below. Section 5.1 describes culture media containing elevated concentrations (*i.e.,* high glycine media) that are suitable for culturing mammalian cells capable of expressing proteins. Section 5.2 describes various glycoproteins (*e.g.*, antibodies) that can be produced in high glycine media. Section 5.3 describes nucleic acids and expression systems for producing glycoproteins. Section 5.4 describes culture methods that can be used to produce glycoproteins. Section 5.5 describes methods of recovering and purifying glycoproteins produced by methods of the disclosure. Section 5.6 describes glycoprotein populations produced by methods of the disclosure. Section 5.7 describes pharmaceutical compositions of glycoproteins produced by methods of the disclosure. Section 5.8 describes methods of treating various diseases using exemplary antibodies produced by methods of the present disclosure.

### 5.1. Cell Culture Media

In one aspect, the present disclosure provides a culture medium suitable for mammalian cell culture comprising high concentrations of glycine. As used herein, the term "culture medium" refers to a medium suitable for growth of mammalian cells in an *in vitro* cell culture. As used herein, the term "cell culture" or "culture" refers to the growth of mammalian cells in an *in vitro* environment. A typical culture medium contains amino acids, vitamins, trace elements, free fatty acids, and an energy source. Additional growth components such as growth factors, nucleosides, lipids, hormones and antibiotics can be included in the culture medium. Furthermore, the medium can include a buffer, an osmolality regulator and a surfactant.

As will be recognized by skilled artisan, the culture and feed media used to culture cells for recombinant protein (*e.g*., antibody) production, as well as other variables such as the feeding schedule, growth rate, temperature, and oxygen levels, can affect the yield and quality of the expressed protein. Methods of optimizing these conditions are within the purview of a skilled artisan; exemplary conditions are set forth in the Exemplary Embodiments of the disclosure. Preferably, cells are adapted to grow in media free of cholesterol-, serum-, and other animal-sourced components; in such instances the culture and feed media preferably include defined chemicals that substitute for such components. Accordingly, the methods of producing recombinant proteins can comprise culturing a recombinant mammalian cell of the present disclosure in a chemically defined medium, free of animal-derived components.

The culture media of the disclosure are high glycine media. As used herein, the term "high glycine medium" refers to a medium in which the concentration of glycine is higher than the concentration of glycine found in typical cell culture media. As shown in Table 1 below, concentrations of glycine in commercial culture media (*e.g*., basal media) range from about 0.1 mM to about 2 mM.

**Table 1: Concentration of glycine in typical commercial culture media**

| **TABLE 1** | |
|---|---|
| **Medium** | **Glycine Concentration (mM)** |
| DMEM | 0.4 |
| DMEM/F12 | 0.25 |
| IMDM | 0.4 |
| MEM, Ham's F12 | 0.1 |
| Medium 199 | 0.67 |
| Medium supplemented with hydrolysate at 5 g/L (assuming 3% glycine) | 2.0 |

The concentration of glycine in the culture media of the disclosure are preferably 5 mM or greater. For instance, the concentration of glycine in the culture media can be from 5 mM to 100 mM (*e.g.,* 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 22 mM, 25 mM, 30 mM or 50 mM). In specific embodiments, the concentration of glycine in the culture medium can be bounded by any of the two foregoing values, such as, but not limited to, a concentration ranging from 5 mM to 10 mM, from 7 mM to 10 mM, from 10 mM to 15 mM, from 12 mM to 17 mM, from 15 mM to 20 mM, from 16 mM to 18 mM, from 10 mM to 30 mM, from 10 mM to 25 mM, from 20 mM to 30 mM, from 10 mM to 50 mM or from 20 mM to 50 mM.

The culture media of the present disclosure can contain a variety of amino acids in addition to glycine. For instance, the culture media can contain other amino acids including alanine, arginine, asparagine, aspartic acid, cysteine, glutamic acid, glutamine, histidine, isoleucine, leucine, lysine, methionine, phenylalanine, proline, serine, threonine, tryptophan, tyrosine and valine. In particular embodiments, the culture media can contain amino acids (other than glycine) present in concentrations that they are normally found in traditional media (*e.g*., basal media). Suitable amino acids have concentrations in the ranges listed in Table 2.

**Table 2: Exemplary concentration ranges of amino acids in the culture media of the present disclosure**

| **TABLE 2** | |
|---|---|
| **Amino Acid** | **Concentration of Amino Acid in the Medium (mM)** |
| Alanine | 0.01-0.07 |
| Arginine | 0.6-1.6 |
| Asparagine | 0.08-1.5 |
| Aspartic Acid | 0.03-0.4 |
| Cysteine | 0.1-0.9 |
| Glutamic Acid | 0.03-0.1 |
| Glutamine | 2-12 |
| Glycine | 8-35 |
| Histidine | 0.09-0.7 |
| Isoleucine | 0.9-1.7 |
| Leucine | 1-1.8 |
| Lysine | 0.8-1.6 |
| Methionine | 0.1-0.5 |
| Phenylalanine | 0.2-1 |
| Proline | 0.5-4 |
| Serine | 0.1-0.8 |
| Threonine | 0.7-1.5 |
| Tryptophan | 0.08-0.3 |
| Tyrosine | 0.2-3 |
| Valine | 0.8-1.6 |

In certain embodiments, the disclosure provides a culture medium in which glycine is at a concentration from 5 mM to 30 mM, and in which one or more amino acids other than glycine have concentrations at or below the concentrations listed in Table 3. For instance, two, three, four, five, six, seven, eight, nine, ten or eleven amino acids can have concentrations at or below the concentrations at or below the concentrations listed in Table 3. In one embodiment, the concentration of lysine in the culture medium is between 0.5 mM and 5.0 mM. Other amino acids not identified in Table 2 can be present in the culture medium, for instance, at concentrations in the ranges listed in Table 2.

**Table 3: Exemplary maximum concentrations of particular amino acids in culture media of certain embodiments of the present disclosure**

| **TABLE 3** | | |
|---|---|---|
| **Amino Acid** | **Concentration of Amino Acid in Medium (mM)** | **Concentration of Amino Acid in Medium (mg/L)** |
| Asparagine | 2 mM | 264 |
| Aspartic Acid | 2 mM | 266 |
| Isoleucine | 2 mM | 262 |
| Leucine | 2.5 mM | 328 |
| Lysine | 6 mM | 876 |
| Methionine | 0.45 mM | 67 |
| Serine | 4 mM | 420 |
| Threonine | 4 mM | 476 |
| Tryptophan | 0.3 mM | 61 |
| Tyrosine | 2.5 mM | 145 |
| Valine | 1.8 mM | 211 |

In other aspects, the disclosure provides a culture medium in which glycine is at a concentration from 5 mM to 30 mM, and in which one or more amino acids other than glycine have concentrations at or below the concentrations listed in Table 4. For instance, two, three, four, five, six, seven, eight, nine, ten or eleven amino acids can have concentrations at or below the concentrations at or below the concentrations listed in Table 4. Other amino acids not identified in Table 3 can be present in the culture medium, for instance, at concentrations in the ranges listed in Table 2.

**Table 4: Exemplary maximum concentrations of particular amino acids in the culture media of certain embodiments of the present disclosure**

| **TABLE 4** | | |
|---|---|---|
| **Amino Acid** | **Concentration of Amino Acid in Medium (mM)** | **Concentration of Amino Acid in Medium (mg/L)** |
| Asparagine | 1.5 mM | 198 |
| Aspartic Acid | 1.5 mM | 200 |
| Isoleucine | 1.5 mM | 197 |
| Leucine | 2 mM | 262 |
| Lysine | 4 mM | 584 |
| Methionine | 0.4 mM | 60 |
| Serine | 2 mM | 210 |
| Threonine | 2 mM | 238 |
| Tryptophan | 0.2 mM | 41 |
| Tyrosine | 1.5 mM | 128 |
| Valine | 1.5 mM | 178 |

Additionally, the culture media of the present disclosure can contain a variety of vitamins Typical vitamins include, but are not limited to, vitamin B-12, biotin, choline, folic acid, nicacinamide, calcium panththenate, pyridoxal hydrochloride, riboflavin and thiamine hydrochloride. The vitamins can suitably be used in the concentration ranges shown in Table 5.

**Table 5: Exemplary concentrations of vitamins in the culture media of the present disclosure**

| **TABLE 5** | |
|---|---|
| **Vitamin** | **Concentration of Vitamin in the Medium (mM)** |
| Vitamin B-12 | 0.00013-0.002 |
| Biotin | 0.00001-0.01 |
| Choline | 0.016-1.3 |
| Folic Acid | 0.0015-0.02 |
| Niacinamide | 0.002-0.07 |
| Calcium pantothenate | 0.012-0.02 |
| Pyridoxal hydrochloride | 0-0.04 |
| Riboflavin | 0.00015-0.0023 |
| Thiamine hydrochloride | 0-0.3 |

Additionally, the cell culture media of the present disclosure can contain a variety of trace elements. Typical trace elements include, but are not limited to, calcium chloride anhydrous, magnesium chloride anhydrous, magnesium sulfate anhydrous, potassium chloride and sodium phosphate dibasic anhydrous. The trace elements can suitably be used in the culture media in concentration ranges shown in Table 6.

**Table 6: Exemplary concentrations of trace elements in culture media of the present disclosure**

| **TABLE 6** | |
|---|---|
| **Trace Element** | **Concentration of Trace Element in the Medium (mM)** |
| Calcium chloride anhydrous | 0.2-1.1 |
| Magnesium chloride anhydrous | 0.1-1. |
| Magnesium sulfate anhydrous | 0.1-1.1 |
| Potassium Chloride | 2-8 |
| Sodium phosphate dibasic anhydrous | 0.5-5 |

The culture media can also include at least one energy source. Examples of energy sources that may be included in the cell culture include glucose, mannose, galactose, maltose and fructose. Glucose can be added to the culture medium at a concentration ranging from 5 g/L to 20 g/L.

The culture media of the present disclosure can also contain a buffer. A buffer is used to maintain the cell culture at a suitable pH. Buffers that can be used in the culture medium include but are not limited to HEPES, phosphate buffer (*e.g*., mono- and di-basic sodium phosphate) and sodium carbonate. The buffer can be used to maintain the pH of the culture medium in an acceptable range, typically at a pH ranging from 6.5 to 7.5 and more typically at a pH ranging from 6.8 to 7.2.

The culture media can contain salts to regulate osmolality. For instance, an osmolality regulator is typically added to maintain the osmolality of the culture medium in a range between 250-400 milli-Osmols (mOsm), and more typically between 270-350 mOsM. Characteristic osmolality regulators include salts such as sodium chloride or potassium chloride. It will be appreciated by one of skill in the art that high glycine amounts in a culture medium will affect the osmolality of the medium. Accordingly, the osmolality of the culture medium can be maintained in an appropriate range by reducing the amount of osmolality regulator added to the medium after accounting for the osmolality contributed by the high glycine levels.

The culture media of the present disclosure can also contain a lipid factor. Typical lipid factors, include, but are not limited to lipoic acid, choline chloride, oleic acid, and phosphatidylcholine.

### 5.2. Glycoproteins

The present disclosure provides glycoproteins enriched in non-fucosylated glycoforms. Section 5.2.1 describes antibodies that can be produced by methods of the present disclosure. Section 5.2.2 describes other types of glycoproteins than can be produced by methods of the disclosure.

### 5.2.1. Antibodies

Antibodies (Abs) and immunoglobulins (Igs) are glycoproteins having the same structural characteristics. While antibodies exhibit binding specificity to a specific target, immunoglobulins include both antibodies and other antibody-like molecules which lack target specificity. Native antibodies and immunoglobulins are usually heterotetrameric glycoproteins of about 150,000 daltons, composed of two identical light (L) chains and two identical heavy (H) chains. Each heavy chain has at one end a variable domain (VH) followed by a number of constant domains. Each light chain has a variable domain at one end (VL) and a constant domain at its other end.

The present disclosure provides antibodies enriched in non-fucosylated glycoforms. Unless indicated otherwise, the term "antibody" (Ab) refers to an immunoglobulin molecule that specifically binds to, or is immunologically reactive with, a particular antigen, and includes polyclonal, monoclonal, genetically engineered and otherwise modified forms of antibodies, including but not limited to chimeric antibodies, humanized antibodies, heteroconjugate antibodies (*e.g*., bispecific antibodies, diabodies, triabodies, and tetrabodies), and antigen binding fragments of antibodies, including *e*.*g*., Fab', F(ab')₂, Fab, Fv, rIgG, and scFv fragments. Moreover, unless otherwise indicated, the term "monoclonal antibody" (mAb) is meant to include both intact molecules, as well as, antibody fragments (such as, for example, Fab and F(ab')₂ fragments) which are capable of specifically binding to a protein. Fab and F(ab')₂ fragments lack the Fc fragment of intact antibody, clear more rapidly from the circulation of the animal, and may have less non-specific tissue binding than an intact antibody (Wahl *et al.,* 1983, J. Nucl. Med.24:316). The term "scFv" refers to a single chain Fv antibody in which the variable domains of the heavy chain and the light chain from a traditional antibody have been joined to form one chain.

References to "VH" refer to the variable region of an immunoglobulin heavy chain of an antibody, including the heavy chain of an Fv, scFv, or Fab. References to "VL" refer to the variable region of an immunoglobulin light chain, including the light chain of an Fv, scFv, dsFv or Fab.

Both the light chain and the heavy chain variable domains have complementarity determining regions (CDRs), also known as hypervariable regions. The more highly conserved portions of variable domains are called the framework (FR). As is known in the art, the amino acid position/boundary delineating a hypervariable region of an antibody can vary, depending on the context and the various definitions known in the art. Some positions within a variable domain may be viewed as hybrid hypervariable positions in that these positions can be deemed to be within a hypervariable region under one set of criteria while being deemed to be outside a hypervariable region under a different set of criteria. One or more of these positions can also be found in extended hypervariable regions. The CDRs in each chain are held together in close proximity by the FR regions and, with the CDRs from the other chain, contribute to the formation of the target binding site of antibodies (see Kabat et al., Sequences of Proteins of Immunological Interest (National Institute of Health, Bethesda, Md. 1987). As used herein, numbering of immunoglobulin amino acid residues is done according to the immunoglobulin amino acid residue numbering system of Kabat *et al.,* unless otherwise indicated.

Antibody fragments can also be produced utilizing the compositions and methods of the disclosure. The term "antibody fragment" refers to a portion of a full-length antibody, generally the target binding or variable region. Examples of antibody fragments include Fab, Fab', F(ab')₂ and Fv fragments. An "Fv" fragment is the minimum antibody fragment which contains a complete target recognition and binding site. This region consists of a dimer of one heavy and one light chain variable domain in a tight, non-covalent association (VH -VL dimer). It is in this configuration that the three CDRs of each variable domain interact to define a target binding site on the surface of the VH -VL dimer. Often, the six CDRs confer target binding specificity to the antibody. However, in some instances even a single variable domain (or half of an Fv comprising only three CDRs specific for a target) can have the ability to recognize and bind target. "Single-chain Fv" or "scFv" antibody fragments comprise the VH and VL domains of an antibody in a single polypeptide chain. Generally, the Fv polypeptide further comprises a polypeptide linker between the VH and VL domains which enables the scFv to form the desired structure for target binding. "Single domain antibodies" are composed of a single VH or VL domains which exhibit sufficient affinity to the target. In a specific embodiment, the single domain antibody is a camelized antibody (see, *e*.*g*., Riechmann, 1999, Journal of Immunological Methods 231:25-38).

The Fab fragment contains the constant domain of the light chain and the first constant domain (CH₁) of the heavy chain. Fab' fragments differ from Fab fragments by the addition of a few residues at the carboxyl terminus of the heavy chain CH₁ domain including one or more cysteines from the antibody hinge region. F(ab') fragments are produced by cleavage of the disulfide bond at the hinge cysteines of the F(ab')₂ pepsin digestion product. Additional chemical couplings of antibody fragments are known to those of ordinary skill in the art.

In certain embodiments, the antibodies of the disclosure are monoclonal antibodies. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies useful with the present disclosure can be prepared using a wide variety of techniques known in the art including the use of hybridoma, recombinant, and phage display technologies, or a combination thereof. The antibodies of the disclosure include chimeric, primatized, humanized, or human antibodies.

The antibodies produced utilizing the methods and compositions of the disclosure can be chimeric antibodies. The term "chimeric" antibody as used herein refers to an antibody having variable sequences derived from a non-human immunoglobulin, such as rat or mouse antibody, and human immunoglobulin constant regions, typically chosen from a human immunoglobulin template. Methods for producing chimeric antibodies are known in the art. See, *e*.*g*., Morrison, 1985, Science 229(4719):1202-7; Oi et al., 1986, BioTechniques 4:214-221; Gillies et al., 1985, J. Immunol. Methods 125:191-202; U.S. Pat. Nos. 5,807,715; 4,816,567; and 4,816397.

The antibodies produced utilizing the methods and compositions of the disclosure can be humanized. "Humanized" forms of non-human (*e.g*., murine) antibodies are chimeric immunoglobulins, immunoglobulin chains or fragments thereof (such as Fv, Fab, Fab', F(ab')₂ or other target-binding subdomains of antibodies) which contain minimal sequences derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody can also comprise at least a portion of an immunoglobulin constant region (Fc), typically that of a human immunoglobulin consensus sequence. Methods of antibody humanization are known in the art. See, *e.g.,* Riechmann et al., 1988, Nature 332:323-7; U.S. Patent Nos: 5,530,101; 5,585,089; 5,693,761; 5,693,762; and 6,180,370 to Queen et al.; EP239400; PCT publication WO 91/09967; U.S. Patent No. 5,225,539; EP592106; EP519596; Padlan, 1991, Mol. Immunol., 28:489-498; Studnicka et al., 1994, Prot. Eng. 7:805-814; Roguska et al., 1994, Proc. Natl. Acad. Sci. 91:969-973; and U.S. Patent No. 5,565,332.

The antibodies produced utilizing the methods and compositions of the disclosure can be human antibodies. Completely "human" antibodies can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods using antibody libraries derived from human immunoglobulin sequences. See U.S. Patent Nos. 4,444,887 and 4,716,111; and PCT publications WO 98/46645; WO 98/50433; WO 98/24893; WO 98/16654; WO 96/34096; WO 96/33735; and WO 91/10741.

Human antibodies can also be produced using transgenic mice which are incapable of expressing functional endogenous immunoglobulins, but which can express human immunoglobulin genes. See, *e.g.,* PCT publications WO 98/24893; WO 92/01047; WO 96/34096; WO 96/33735; U.S. Patent Nos. 5,413,923; 5,625,126; 5,633,425; 5,569,825; 5,661,016; 5,545,806; 5,814,318; 5,885,793; 5,916,771; and 5,939,598.

In addition, companies such as Medarex (Princeton, NJ), Astellas Pharma (Deerfield, IL), and Regeneron (Tarrytown, NY) can be engaged to provide human antibodies directed against a selected antigen using technology similar to that described above. Completely human antibodies that recognize a selected epitope can be generated using a technique referred to as "guided selection." In this approach a selected non-human monoclonal antibody, *e*.*g*., a mouse antibody, is used to guide the selection of a completely human antibody recognizing the same epitope (Jespers et al., 1988, Biotechnology 12:899-903).

The antibodies produced utilizing the methods and compositions of the disclosure can be primatized. The term "primatized antibody" refers to an antibody comprising monkey variable regions and human constant regions. Methods for producing primatized antibodies are known in the art. See *e.g.,* U.S. Patent Nos. 5,658,570; 5,681,722; and 5,693,780.

The antibodies produced utilizing the methods and compositions of the disclosure can be bispecific antibodies. Bispecific antibodies are monoclonal, often human or humanized, antibodies that have binding specificities for at least two different antigens.

The antibodies produced utilizing the methods and compositions of the disclosure include derivatized antibodies. For example, but not by way of limitation, derivatized antibodies are typically modified by glycosylation, acetylation, pegylation, phosphorylation, amidation, derivatization by known protecting/blocking groups, proteolytic cleavage, linkage to a cellular ligand or other protein, *etc.* Any of numerous chemical modifications can be carried out by known techniques, including, but not limited to, specific chemical cleavage, acetylation, formylation, metabolic synthesis of tunicamycin, *etc.* Additionally, the derivative can contain one or more non-natural amino acids, *e*.*g*., using ambrx technology (see, *e.g.*, Wolfson, 2006, Chem. Biol. 13(10):1011-2).

In yet another embodiment of the disclosure, the antibodies or fragments thereof can be antibodies or antibody fragments whose sequence has been modified to alter at least one constant region-mediated biological effector function relative to the corresponding wild type sequence.

For example, in some embodiments, an antibody of the disclosure can be modified to reduce at least one constant region-mediated biological effector function relative to an unmodified antibody, *e*.*g*., reduced binding to the Fc receptor (FcR). FcR binding can be reduced by mutating the immunoglobulin constant region segment of the antibody at particular regions necessary for FcR interactions (see *e.g.,* Canfield and Morrison, 1991, J. Exp. Med. 173:1483-1491; and Lund et al., 1991, J. Immunol. 147:2657-2662).

In other embodiments, the antibodies produced utilizing the methods and compositions of the disclosure modified to acquire or improve at least one constant region-mediated biological effector function relative to an unmodified antibody, *e.g.,* to enhance FcγR interactions (See, *e.g.,* US 2006/0134709). For example, an antibody with a constant region that binds FcγRIIA, FcγRIIB and/or FcγRIIIA with greater affinity than the corresponding wild type constant region can be produced according to the methods described herein.

Examples of antibodies that can be produced using methods of the present disclosure include, but are not limited to adalimumab, elotuzumab, enavatuzumab, daclizumab, voloxicimab, tositumomab, trastuzumab, istekinumab, abcicimab, besilesomab, etaracizumab, pemtumomab, omalizumab, pertuzumab, natalizumab, sulesomab, tefibazumab, votumumab, motavizumab, oregovomabm, panitumumab, zalutumumab, igovomab, bevacizumab, basiliximab, atlizumab, bectumomab, belimumab, alemtuzumab, nimotuzumab, mepolizumab, altumomab, ranibizumab, rituximab, palivizumab, gemtuzumab, golimumab, fontolizumab, nofetumomab, ofatumumab, arcitumomab, cetuximab, imciromab, certolizumab, rovelizumab, ruplizumab, ipilimumab, labetuzumab, catumaxomab, canakinumab, denosumab, eculizumab, fanolesomab, efalizumab, infliximab, edrecolomab, efungumab, girentuximab, ertumaxomab and toclizumab.

### 5.2.2. Other Glycoproteins

The methods of the disclosure can be used to produce glycoproteins of any protein, including proteins for therapeutic purposes. Examples of proteins that can be produced in accordance with the present disclosure include, but are not limited to growth factors such as eyhtropoietin (EPO), human chronic gonadotropin (hCG), granulocyte colony-stimulating factor (GCSF), antithrombin III, interleukin 1, interleukin 2, interleukin 6, human chorionic gonadotropin (hCG), antitrombin III, interferon alpha, interferon beta, interferon gamma, coagulation factors such as factor VIIIm factor IX and human protein C, epidermal growth factor, growth hormone-releasing factor, epidermal growth factor, angiostatin, vascular endothelial growth factor-2 and plasminogen activator.

### 5.3. Nucleic Acids and Expression Systems

Standard recombinant DNA methodologies such as those described in Molecular Cloning; A Laboratory Manual, Second Edition (Sambrook, Fritsch and Maniatis (eds), Cold Spring Harbor, N. Y., 1989), Current Protocols in Molecular Biology (Ausubel, F.M. et al., eds., Greene Publishing Associates, 1989) and in U.S. Patent No. 4,816,397 can be used to produce recombinant mammalian cells suitable for producing glycoproteins in accordance with methods of the disclosure.

Glycoprotein sequences are well-known to those of skill in the art. In embodiments where the glycoprotein is an antibody, recombinant techniques can be used to obtain antibody heavy and light chain genes, incorporate these genes into recombinant expression vectors and introduce the vectors into host cells. To express an antibody recombinantly, a host cell is transfected with one or more recombinant expression vectors carrying DNA fragments encoding the immunoglobulin light and heavy chains of the antibody such that the light and heavy chains are expressed in the host cell and, optionally, secreted into the medium in which the host cells are cultured, from which medium the antibodies can be recovered. To generate nucleic acids encoding antibodies, DNA fragments encoding the light and heavy chain variable regions are first obtained. These DNAs can be obtained by amplification and modification of germline DNA or cDNA encoding light and heavy chain variable sequences, for example using the polymerase chain reaction (PCR). Germline DNA sequences for human heavy and light chain variable region genes are known in the art (See *e.g.,* the "VBASE" human germline sequence database; see also Kabat, E. A. et al., 1991, Sequences of Proteins of Immunological Interest, Fifth Edition, U.S. Department of Health and Human Services, NIH Publication No. 91-3242; Tomlinson et al., 1992, J. Mol. Biol. 22T:116-198; and Cox et al., 1994, Eur. J. Immunol. 24:827-836.

A DNA fragment encoding the heavy or light chain variable region of the antibody can be synthesized and used as a template for mutagenesis to generate a variant as described herein using routine mutagenesis techniques; alternatively, a DNA fragment encoding the variant can be directly synthesized.

Once DNA fragments encoding the antibody are obtained, these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked," as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH₁, CH₂, CH₃ and, optionally, CH₄). The sequences of human heavy chain constant region genes are known in the art (see *e.g.,* Kabat, E.A., et al., 1991, Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG₁, IgG₂, IgG₃, IgG₄, IgA, IgE, IgM or IgD constant region, but in certain embodiments is an IgG₁ or IgG₄ constant region. For a Fab fragment heavy chain gene, the VH-encoding DNA can be operatively linked to another DNA molecule encoding only the heavy chain CH₁ constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (See *e.g.,* Kabat, E. A., et al., 1991, Sequences of Proteins of Immunological Interest, Fifth Edition (U.S. Department of Health and Human Services, NIH Publication No. 91-3242)) and DNA fragments encompassing these regions can be obtained by standard PCR amplification. The light chain constant region can be a kappa or lambda constant region, but in certain embodiments is a kappa constant region. To create a scFv gene, the VH- and VL-encoding DNA fragments are operatively linked to another fragment encoding a flexible linker, *e*.*g*., encoding the amino acid sequence (Gly₄∼Ser)₃ (SEQ ID NO:21), such that the VH and VL sequences can be expressed as a contiguous single-chain protein, with the VL and VH regions joined by the flexible linker (See *e.g.,* Bird et al., 1988, Science 242:423-426; Huston et al., 1988, Proc. Natl. Acad. Sci. USA 85:5879-5883; McCafferty et al., 1990, Nature 348:552-554).

To express glycoproteins, DNAs encoding the glycoprotein are inserted into expression vectors such that the genes are operatively linked to transcriptional and translational control sequences. In this context, the term "operatively linked" is intended to mean that a glycoprotein encoding a nucleic acid is ligated into a vector such that transcriptional and translational control sequences within the vector serve their intended function of regulating the transcription and translation of the glycoprotein coding sequence. The expression vector and expression control sequences are chosen to be compatible with the expression host cell used. Where the glycoprotein is an antibody, the light chain gene and the antibody heavy chain gene can be inserted into separate vectors or, more typically, both genes are inserted into the same expression vector.

The glycoprotein encoding nucleic acids can be inserted into the expression vector by standard methods (*e.g.*, ligation of complementary restriction sites on the antibody gene fragment and vector, or blunt end ligation if no restriction sites are present). Where the glycoprotein is an antibody, prior to insertion of the antibody light or heavy chain sequences, the expression vector can already carry antibody constant region sequences. For example, one approach to converting the antibody VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.,* a signal peptide from a non-immunoglobulin protein).

The recombinant expression vectors can carry regulatory sequences that control the expression of the glycoprotein coding sequences in a host cell. The term "regulatory sequence" is intended to include promoters, enhancers and other expression control elements (*e.g*., polyadenylation signals) that control the transcription or translation of the antibody chain genes. Such regulatory sequences are described, for example, in Goeddel, Gene Expression Technology: Methods in Enzymology 185 (Academic Press, San Diego, CA, 1990). It will be appreciated by those skilled in the art that the design of the expression vector, including the selection of regulatory sequences may depend on such factors as the choice of the host cell to be transformed, the level of expression of protein desired, *etc.* Suitable regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e.g.,* the adenovirus major late promoter (AdMLP)) and polyoma. For further description of viral regulatory elements, and sequences thereof, see *e.g.,* U.S. Patent No. 5,168,062 by Stinski, U.S. Patent No. 4,510,245 by Bell *et al.,* and U.S. Patent No. 4,968,615 by Schaffner et al.

The recombinant expression vectors can carry additional sequences, such as sequences that regulate replication of the vector in host cells (*e.g*., origins of replication) and selectable marker genes. The selectable marker gene facilitates selection of host cells into which the vector has been introduced (see *e.g.,* U.S. Patents Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Suitable selectable marker genes include the dihydrofolate reductase (DHFR) gene (for use in DHFR⁻ host cells with methotrexate selection/amplification) and the neo gene (for G418 selection). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a mammalian host cell.

The glycoproteins of the disclosure can be expressed in mammalian host cells, for optimal secretion of a properly folded and immunologically active protein. Exemplary mammalian host cells for expressing the recombinant antibodies of the disclosure include Chinese Hamster Ovary (CHO cells) (including DHFR⁻ CHO cells, described in Urlaub and Chasin, 1980, Proc. Natl. Acad. Sci. USA 77:4216-4220, used with a DHFR selectable marker, *e*.*g*., as described in Kaufman and Sharp, 1982, Mol. Biol. 159:601-621), NSO myeloma cells, COS cells, SP2/0 cells, EB66® cells, and PER.C6® cells. When recombinant expression vectors encoding glycoproteins are introduced into mammalian host cells, the glycoproteins are produced by culturing the host cells for a period of time sufficient to allow for expression of the glycoprotein in the host cells or secretion of the glycoprotein into the culture medium in which the host cells are grown. Suitable culturing techniques are described in Section 5.4. Glycoproteins can be recovered from the culture medium using standard protein purification methods, for example, as described in Section 5.5.

### 5.4. Culture Methods

The disclosure provides methods for producing glycoproteins (*e.g*., antibodies) by culturing mammalian cells engineered to express the glycoproteins in a high glycine culture medium, such as a culture medium described in Section 5.1. Methods of the disclosure provide glycoproteins with reduced fucosylated glycoforms as compared to glycoproteins produced in traditional culture media.

The recombinant mammalian cells described in Section 5.3 can be cultured in suspension, such as by shake flask cultivation in small-scale or large-scale fermentation (including continuous, batch, fed-batch, or solid state fermentations) in laboratory or industrial fermentors performed in a suitable medium and under conditions allowing the glycoprotein to be expressed and/or isolated. Alternatively, the mammalian cells can be cultured while attached to a solid substrate.

In particular embodiments, the recombinant mammalian cells are added to the basal medium at an initial cell density in the range of 0.5 - 5 x 10⁵ cells/mL and more typically in the range of 1.5 - 2.5 x 10⁵ cells/mL. The cultures are generally harvested for approximately 8 days to 20 days following inoculation, and more typically between 10 days to 14 days following inoculation.

Following inoculation of the mammalian cells, the growth of the cells can be promoted by addition of nutrients according to a pre-determined feeding schedule. Addition of the feed medium to the culture can be through processes known in the art such as continuous culture, batch culture and fed-batch culture. To support the propagation of cells, a feed solution can be added to the culture at intermittent times following inoculation. As used herein, the term "feed" refers to nutrients added to the culture after inoculation. The term "feed solution" or "feed media" refers to a solution containing feed that is added to the culture following inoculation.

### 5.5. Recovery and Purification

Techniques for recovering and purifying expressed protein are well known in the art and can be tailored to the particular glycoprotein(s) being expressed by the recombinant mammalian cell. Glycoproteins can be recovered from the culture medium and or cell lysates. In embodiments where the method is directed to producing a secreted glycoprotein, the glycoprotein can be recovered from the culture medium. Proteins may be recovered or purified from culture media by a variety of procedures known in the art including but not limited to, centrifugation, filtration, extraction, spray-drying, evaporation, or precipitation. The recovered glycoprotein may then be further purified by a variety of procedures known in the art including, but not limited to, chromatography (*e.g*., ion exchange, affinity, hydrophobic, chromatofocusing, and size exclusion), electrophoretic procedures (*e*.*g*., preparative isoelectric focusing (IEF), differential solubility (*e.g.*, ammonium sulfate precipitation), or extraction (see, *e*.*g*., Protein Purification, J.-C. Janson and Lars Ryden, editors, VCH Publishers, New York, 1989).

In embodiments where the glycoprotein is an antibody, the antibody can be purified by a process that utilizes Protein A affinity chromatography in conjunction with strong anion exchange (Q-Sepharose) chromatography and weak cation exchange (CM-650M) chromatography, permitting continuous flow processing without dilution of process intermediate. This method for obtaining purified antibody can entail the following steps:
(i) protein A affinity chromatography to isolate antibody from other cell culture components;
(ii) low pH viral inactivation;
(iii) strong anion exchange chromatography to remove DNA;
(iv) weak cation exchange chromatography to reduce aggregates; and
(v) filtration to remove viruses.
Steps (ii) - (v) can be carried out in any order.

Once isolated, an antibody can, if desired, be further purified, *e*.*g*., by high performance liquid chromatography (see, *e*.*g*., Fisher, Laboratory Techniques In Biochemistry And Molecular Biology (Work and Burdon, eds., Elsevier, 1980), or by gel filtration chromatography on a Superdex^{™} 75 column (Pharmacia Biotech AB, Uppsala, Sweden).

### 5.6. Protein Glycoforms

Post-translational modification of a protein by glycosylation can influence the biochemical and physical properties of the protein. For instance, the properties of an antibody can be influenced by the glycosylation at both the Fc and the Fab regions of the antibody. "Glycosylation" refers to the covalent modification of proteins (*e.g.,* antibodies) with carbohydrates (*e.g.,* oligosaccharide chains). Typical sugars in glycoproteins include mannose, glucose, galactose, fucose, N-acetylgalactosamine (GalNAc), N-acetylglucosamine (GlcNAc), and sialic acid.

The individual glycosylated molecules (*e.g*., glycosylated antibodies) are referred to herein as glycoforms. The "glycosylation profile" refers to the set of glycoforms in a particular sample. Glycosylation generally refers to N-linked glycosylation or O-linked glycosylation. The term "N-linked glycosylation" refers to the covalent linkage of an oligosaccharide chain to an asparagine residue of a protein, thereby forming N-linked oligosaccharides. N-linked oligosaccharides are also referred to as N-linked glycans. The term "O-linked glycosylation" refers to the covalent linkage of an oligosaccharide chain to the hydroxyl group of a serine or threonine residue of a protein, thereby forming O-linked oligosaccharides. O-linked oligosaccharides are also referred to as O-linked glycans.

N-linked glycans are further characterized by the number of galactose residues at their terminal ends, whether or not they contain a fucose attached to the N-linked-N-acetyl glucosamine. N-linked glycans that have a fucose molecule attached to the N-linked N-acetyl glucosamine are referred to herein as fucosylated N-linked glycans. Proteins with fucosylated N-linked glycans are referred to herein as fucosylated glycoforms. The characteristics of four different fucosylated N-linked glycans are listed below (also see **FIGURE 1****):**
- G0: Biantennary (*i.e.,* having two antennas) core structure with fucose attached to the N-linked N-acetyl glucosamine and one N-acetyl glucosamine on each branch of the core structure.
- G1: Biantennary core structure with fucose attached to the N-linked N-acetyl glucosamine, one N-acetyl glucosamine on each branch of the core structure and terminal galactose on one branch of the core structure.
- G2: Biantennary core structure with fucose attached to the N-linked N-acetyl glucosamine, one N-acetyl glucosamine and one terminal galactose on each branch of the core structure.
- G0-GlcNAc: Biantennary core structure with fucose attached to the N-linked N-acetyl glucosamine, one N-acetyl glucosamine on one branch of the core structure and no terminal galactose.

N-linked glycans that do not contain a fucose molecule are referred to as non-fucosylated glycans. Proteins with non-fucosylated glycans are referred to as non-fucosylated glycoforms. For instance, a G0-GlcNAc-Fucose N-linked glycan has a biantennary core structure without fucose attached to the N-linked N-acetyl glucosamine, one N-acetyl glucosamine on one branch of the core structure and no terminal galactose. An antibody containing a G0-GlcNAc-Fucose N-linked glycan is referred to herein as a G0-GlcNAc-Fucose N-linked glycoform.

N-linked glycosylation profiles can be assessed by various methods. For example, N-linked glycosylation profiles can be assessed by digesting the antibody with trypsin and analyzing the resulting peptide mixture using reverse-phase chromatography with mass spectrometric detection and quantitation of the various glycopeptides. In a specific method, the digested peptides are analyzed using a YMC-Pack ODS-AQ, 5 um particle size, 120 Angstrom pore size, 2.0 mm x 250 mm reverse phase column (YMC Co., Ltd, catalog number AQ12S05-2502WT) and the eluted peptides are detected and quantified using a Thermo LCQ Deca XP+ mass spectrometer (Thermo Finnigan). The relative abundance of each glycoform is determined based on the mass spectrometric extracted ion chromatogram peak area of the corresponding glycopeptides relative to the sum of the peak areas of all observed glycopeptides.

Alternatively, N-linked glycosylation profiles can be assessed by cleaving the N-linked oligosaccharides with amidase PNGase F, derivatizing the oligosaccharides with a fluorescent label and analyzing the resultant mixture via normal phase HPLC with fluorescent detection. In a specific method, derivatized, cleaved N-linked glycans are resolved at 50°C on a 250 x 4.6 mm polymeric-amine bonded Asahipak Amino NH₂P-504E column (5 µm particle size, Phenomenex, cat. No. CHO-2628).

Studies of the glycosylation profile of IgG have revealed that IgG carries at least 30 different N-linked oligosaccharides. See Dwek et al., 1995, J. Anat. 187:279-292. N-linked glycosylation cites are found on both the Fc region and the Fab regions of many antibodies. N-linked oligosaccharides without any sialic acid groups are often referred to as neutral N-linked oligosaccharides. N-linked oligosaccharides with one or two sialic acid groups at the non-reducing sugar terminus of the oligosaccharide are referred to as monosialyted or disialylated N-linked oligosaccharides, respectively. With respect to IgG, Fc glycosylation is characterized by a low incidence of monosialyted and disialylated structures while Fab glycosylation is characterized by a high incidence of monosialyted and disialylated structures.

Additional N-linked glycans have been identified in the Fc region of antibodies. For instance, glycans containing high amounts of mannose characterized by glycans that only contain two N-acetyl glucosamines with the remaining residues containing mannose are referred to as "high mannose glycans". The mannose residues are covalently attached to a GlcNAc at the 2-position of the oligosaccharide. One example of a high mannose five glycan is "Mannose5 glycan". Mannose 5 glycans contain five mannose residues. Proteins with Mannose5 glycans are referred to herein as "Mannose5 glycoforms". The high mannose glycans are examples of non-fucosylated glycans.

The present disclosure provides methods of producing antibodies enriched in glycoforms not containing fucose (*i*.*e*., non-fucosylated glycoforms). Examples of non-fucosylated glycoforms include, but are not limited to G0-GLcNAc- Fucose glycoforms and Mannose5 glycoforms. Antibodies lacking fucose have been correlated with enhanced ADCC (antibody-dependent cellular cytotoxicity) activity. Lymphocytes, particularly Natural Killer (NK) cells, contain surface-bound receptors that are capable of binding to the Fc region of antibodies. For instance, the human CD16a receptor on NK cells binds to the Fc region of an antibody bound to a target cell, thereby initiating killing of the target cell. Without being bound by theory, it is believed that non-fucosylated glycoforms produced in accordance with methods of the present disclosure display higher binding affinity to the CD16a receptor on NK cells than glycoforms containing fucose (*i*.*e*., fucosylated glycoforms), which is responsible for the increased ADCC activity.

Antibodies produced in high glycine media as described herein contain significantly more non-fucosylated glycoforms than antibodies produced in conventional media. As described in Section 5.3, the total amount of glycans lacking fucose generally increases as the concentration of glycine in the culture medium increases from 2 mM to 30 mM. In particular, the percentage of G0-GlcNAc-Fucose and Mannose5 glycans increase when NS0 cells are cultured in the culture media produced in accordance with the present disclosure. While the amount of non-fucosylated antibodies produced in culture media of the present disclosure is dependent on the AOI, the percentage increase of glycans lacking fucose is generally at least 120% relative to antibodies produced in conventional media (*i.e.,* media with glycine concentrations of 2 mM or less). For instance, the percent increase of total glycans lacking fucose on antibodies produced in culture media of the present disclosure can be 120%, 150%, 180%, 200%, 250%, 300% or 400% relative to antibodies produced in conventional media. In specific embodiments, the percent increase of total glycans lacking fucose on antibodies produced by methods of the disclosure relative to antibodies produced in conventional basal media be bounded by any of the two foregoing values, such as, but not limited to, 120-150%, 150-180%, 150-200%, or 200-300%.

The present disclosure further provides compositions comprising particular antibodies of interest with altered glycosylation profiles relative to antibodies produced in conventional media. In particular, the antibodies produced by the methods of the present disclosure have a greater percentage of non-fucosylated glycoforms than antibodies produced in conventional media. As a result, antibodies produced by methods of the present disclosure generally display higher ADCC activity than antibodies produced in conventional media and show higher binding to the CD16a receptor on NK cells.

In one particular embodiment, the methods of the present disclosure can be used to produce a monoclonal antibody having a VH region of the amino acid sequence SEQ ID NO:1 and a VL region of the amino acid sequence SEQ ID NO:2. The monoclonal antibody, referred to as elotuzumab (HuLuc 63), was disclosed in U.S. Patent Publication No. 2006/0024296 as having VH and VL regions of SEQ ID NO:41 and SEQ ID NO:44, respectively.
Elotuzumab has a full heavy chain amino acid sequence of SEQ ID NO:3 and a full light chain amino acid sequence of SEQ ID NO:4. Elotuzumab exhibits *in vitro* antibody-dependent cellular cytotoxicity (ADCC) in primary myeloma cells and *in vivo* anti-tumor activity (Hsi et al., (2008) Clin. Cancer Res. 14(9):2775).

The disclosure provides a composition comprising a population of elotuzumab molecules, wherein at least 4% of the glycans lack fucose, which is produced in accordance with the cell culturing process of the present disclosure. For instance, at least 4%, at least 5%, at least 6%, at least 7% or at least 8% of the glycans on elotuzumab lack fucose. In particular embodiments, the non-fucosylated glycans of elotuzumab produced in accordance with the cell culturing process of the present disclosure include, but are not limited to N-linked glycans such as G0-GlcNAC-Fucose and Mannose5 glycans. In these embodiments, at least 4% of the N-linked glycans lack fucose. In other embodiments, the disclosure provides elotuzumab molecules having at least 2.5% of the total glycans present as Mannose5 glycans. For instance, at least 3%, at least 4% or at least 5% of the glycans on elotuzumab can be present as Mannose5 glycans.

In another embodiment, the methods of the present disclosure can be used to produce a monoclonal antibody having a VH region of the amino acid sequence SEQ ID NO:5 and a VL region of the amino acid sequence SEQ ID NO:6. The monoclonal antibody, referred to as daclizumab, is a humanized IgG₁ antibody that specifically binds the alpha subunit (also referred to as CD25 or Tac) of the human interleukin-2 receptor (IL-2R), which is an important mediator of lymphocyte activation. Daclizumab has a full heavy chain amino acid sequence of SEQ ID NO:7 and a full light chain amino acid sequence of SEQ ID NO:8.

The disclosure provides a composition comprising a population of daclizumab molecules, wherein at least 3% of glycans lack fucose, which is produced in accordance with the cell culturing process of the present disclosure. For instance, at least 3%, at least 4%, at least 5%, at least 6%, at least 7% or at least 8% of the glycans on daclizumab lack fucose. In particular embodiments, the non-fucosylated glycans of daclizumab produced in accordance with the cell culturing process of the present disclosure include, but are not limited to N-linked glycans such as G0-GlcNAC-Fucose and Mannose5 glycans. In these embodiments, at least 3% of the N-linked glycans lack fucose. In other embodiments, the disclosure provides daclizumab molecules having at least 2 % of the total glycans present as Mannose5 glycans. For instance, at least 3%, at least 4% or at least 5% of the glycans on daclizumab can be present as Mannose5 glycans.

In another embodiment, the methods of the present disclosure can be used to produce a monoclonal antibody having a VH region of the amino acid sequence SEQ ID NO:9 and a VL region of the amino acid sequence SEQ ID NO:10. The monoclonal antibody, referred to as voloxicimab, is a high-affinity IgG4 chimeric (82% human, 18% murine) monoclonal antibody (mAb) that specifically binds to α₅β₁ integrin used for the treatment of a variety of advanced stage tumors. Voloxicimab has a full heavy chain amino acid sequence of SEQ ID NO:11 and a full light chain amino acid sequence of SEQ ID NO:12.

The disclosure provides a composition comprising a population of voloxicimab molecules, wherein at least 1% of the glycans lack fucose, which is produced in accordance with the cell culturing process of the present disclosure. For instance, at least 1% of the glycans on voloxicimab lack fucose. In particular embodiments, the non-fucosylated glycans of voloxicimab produced in accordance with the cell culturing process of the present disclosure include, but are not limited to N-linked glycans such as G0-GlcNAC-Fucose and Mannose5 glycans. In these embodiments, at least 1% of the N-linked glycans lack fucose. In other embodiments, the disclosure provides voloxicimab molecules having at least 0.5% of the total glycans present as Mannose5 glycans.

In another embodiment, the methods of the present disclosure can be used to produce a monoclonal antibody having a VH region of the amino acid sequence SEQ ID NO:13 and a VL region of the amino acid sequence SEQ ID NO:14. The monoclonal antibody, referred to as PDL241, is a humanized IgG₁ antibody that binds to the protein CS1 (but at a different epitope than elotuzumab). PDL241 has a full heavy chain amino acid sequence of SEQ ID NO:15 and a full light chain amino acid sequence of SEQ ID NO:16.

The disclosure provides a composition comprising a population of PDL241 molecules, wherein at least 14% of the glycans lack fucose, which is produced in accordance with the cell culturing process of the present disclosure. For instance, at least 14%, at least 15%, at least 16%, at least 17%, at least 18% or at least 19% of the glycans on PDL241 lack fucose. In particular embodiments, the non-fucosylated glycans of PDL241 produced in accordance with the cell culturing process of the present disclosure include, but are not limited to N-linked glycans such as G0-GlcNAC-Fucose and Mannose5 glycans. In these embodiments, at least 14% of the N-linked glycans lack fucose. In other embodiments, the disclosure provides PDL241 molecules having at least 8 % of the total glycans present as Mannose5 glycans. For instance, at least 9%, at least 10%, at leat 11% or at least 12% of the glycans on PDL241 can be present as Mannose5 glycans.

In another embodiment, the methods of the present disclosure can be used to produce a monoclonal antibody having a VH region of the amino acid sequence SEQ ID NO:17 and a VL region of the amino acid sequence SEQ ID NO:18. The monoclonal antibody, referred to as enavatuzumab (PDL192) is a humanized IgG1 antibody to TweakR that exhibits anti-tumor activity in preclinical models through two mechanisms: direct signaling through TweakR and antibody-dependent cellular cytotoxicity. Enavatuzumab has a full heavy chain amino acid sequence of SEQ ID NO:19 and a full light chain amino acid sequence of SEQ ID NO:20.

The disclosure provides a composition comprising a population of enavatuzumab molecules, wherein at least 5% of the glycans lack fucose, which is produced in accordance with the cell culturing process of the present disclosure. For instance, at least 5%, at least 6%, at least 7%, at least 8%, at least 9% or at least 10% of the glycans on enavatuzumab lack fucose. In particular embodiments, the non-fucosylated glycans of enavatuzumab produced in accordance with the cell culturing process of the present disclosure include, but are not limited to N-linked glycans such as G0-GlcNAC-Fucose and Mannose5 glycans. In these embodiments, at least 5% of the N-linked glycans lack fucose. In other embodiments, the disclosure provides enavatuzumab molecules having at least 2.5% of the total glycans present as Mannose5 glycans. For instance, at least 3%, at least 4% or at least 5% of the glycans on elotuzumab can be present as Mannose5 glycans.

Table 7 below provides the sequences of elotuzumab, daclizumab, voloxicimab, PDL241 and enavatuzumab as identified above.

**Table 7: Sequences of elotuzumab, daclizumab, voloxicimab, PDL241 and enavatuzumab**

| **TABLE 7** | | |
|---|---|---|
| **SEQ ID NO.** | **Description** | **Sequence** |
| 1 | Elotuzumab heavy chain variable region | |
| 2 | Elotuzumab light chain variable region | |
| 3 | Elotuzumab heavy chain full sequence | |
| 4 | Elotuzumab light chain full sequence | |
| 5 | Daclizumab heavy chain variable region | |
| 6 | Daclizumab light chain variable region | |
| 7 | Daclizumab heavy chain full sequence | |
| 8 | Daclizumab light chain full sequence | |
| 9 | Voloxicimab heavy chain variable region | |
| 10 | Voloxicimab light chain variable region | |
| 11 | Voloxicimab heavy chain full sequence | |
| 12 | Voloxicimab light chain full sequence | |
| 13 | PDL241 heavy chain variable region | |
| 14 | PD241 light chain variable region | |
| 15 | PDL241 heavy chain full sequence | |
| 16 | PDL241 light chain full sequence | |
| 17 | Enavatuzumab heavy chain variable region | |
| 18 | Enavatuzumab light chain variable region | |
| 19 | Enavatuzumab heavy chain full sequence | |
| 20 | PDL241 light chain full sequence | |

### 5.7. Pharmaceutical Compositions

The glycoprotein compositions described above can be supplied as part of a sterile, pharmaceutical composition that includes a pharmaceutically acceptable carrier. This composition can be in any suitable form (depending upon the desired method of administering it to a patient). The glycoprotein compositions can be administered to a patient by a variety of routes such as orally, transdermally, subcutaneously, intranasally, intravenously, intramuscularly, intrathecally, topically or locally. The most suitable route for administration in any given case will depend on the particular glycoprotein, the subject, and the nature and severity of the disease and the physical condition of the subject. Typically, the glycoprotein compositions will be administered intravenously or subcutaneously.

Pharmaceutical compositions can be conveniently presented in unit dose forms containing a predetermined amount of a glycoprotein composition of the disclosure per dose. Such a unit can contain for example but without limitation 0.5 mg to 5 g, for example 10 mg to 1 g, or 20 to 50 mg. Pharmaceutically acceptable carriers for use in the disclosure can take a wide variety of forms depending, *e.g.,* on the condition to be treated or route of administration.

Therapeutic formulations of the glycoprotein compositions of the disclosure can be prepared for storage as lyophilized formulations or aqueous solutions by mixing the glycoprotein having the desired degree of purity with optional pharmaceutically-acceptable carriers, excipients or stabilizers typically employed in the art (all of which are referred to herein as "carriers"), *i.e.,* buffering agents, stabilizing agents, preservatives, isotonifiers, non-ionic detergents, antioxidants, and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentration ranging from about 2 mM to about 50 mM. Suitable buffering agents for use with the present disclosure include both organic and inorganic acids and salts thereof such as citrate buffers (*e.g*., monosodium citrate-disodium citrate mixture, citric acid-trisodium citrate mixture, citric acid-monosodium citrate mixture, *etc*.), succinate buffers (*e.g.,* succinic acid-monosodium succinate mixture, succinic acid-sodium hydroxide mixture, succinic acid-disodium succinate mixture, *etc*.), tartrate buffers (*e.g.,* tartaric acid-sodium tartrate mixture, tartaric acid-potassium tartrate mixture, tartaric acid-sodium hydroxide mixture, *etc*.), fumarate buffers (*e.g.,* fumaric acid-monosodium fumarate mixture, fumaric acid-disodium fumarate mixture, monosodium fumarate-disodium fumarate mixture, *etc*.), gluconate buffers (*e.g.,* gluconic acid-sodium glyconate mixture, gluconic acid-sodium hydroxide mixture, gluconic acid-potassium glyuconate mixture, *etc*.), oxalate buffer (*e*.*g*., oxalic acid-sodium oxalate mixture, oxalic acid-sodium hydroxide mixture, oxalic acid-potassium oxalate mixture, *etc*.), lactate buffers (*e.g.,* lactic acid-sodium lactate mixture, lactic acid-sodium hydroxide mixture, lactic acid-potassium lactate mixture, *etc*.) and acetate buffers (*e.g.,* acetic acid-sodium acetate mixture, acetic acid-sodium hydroxide mixture, *etc*.). Additionally, phosphate buffers, histidine buffers and trimethylamine salts such as Tris can be used.

Preservatives can be added to retard microbial growth, and can be added in amounts ranging from 0.2%-1% (w/v). Suitable preservatives for use with the present disclosure include phenol, benzyl alcohol, meta-cresol, methyl paraben, propyl paraben, octadecyldimethylbenzyl ammonium chloride, benzalconium halides (*e.g*., chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions of the present disclosure and include polhydric sugar alcohols, for example trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, *etc*., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (*e.g*., peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the glycoprotein as well as to protect the glycoprotein against agitation-induced aggregation, which also permits the formulation to be exposed to shear surface stressed without causing denaturation of the protein. Suitable non-ionic surfactants include polysorbates (20, 80, *etc*.), polyoxamers (184, 188 *etc*.), Pluronic polyols, polyoxyethylene sorbitan monoethers (TWEEN®-20, TWEEN®-80, *etc*.). Non-ionic surfactants can be present in a range of about 0.05 mg/ml to about 1.0 mg/ml, for example about 0.07 mg/ml to about 0.2 mg/ml.

Additional miscellaneous excipients include bulking agents (*e.g.,* starch), chelating agents (*e.g.,* EDTA), antioxidants (*e.g*., ascorbic acid, methionine, vitamin E), and cosolvents.

### 5.8. Methods of Treatment

### 5.8.1. Elotuzumab and PDL241 Compositions

The elotuzumab and PDL241 compositions described in Section 5.6 are useful for treating a variety of disorders and conditions thought to implicate increased expression of the protein CS1 on neoplastic cells, including, for example, multiple myeloma. Specific patient populations, formulations, modes of administration and dosage amounts and schedules useful for treating or preventing multiple myeloma are described in U.S. Patent No. 7,709,610. All of these formulations, modes of administration, dosing amounts and schedules, as well as disclosed specific patient populations and combination therapies, are equally suited to the elotuzumab and PDL241 compositions described herein.

The elotuzumab and PDL241compositions and formulations described herein are administered in amounts that provide therapeutic benefit. Therapeutic benefit includes, but is not limited, to treatment of the underlying disorder. Therapeutic benefit may also include improving or ameliorating symptoms or side effects of a particular disease as assessed using standard diagnostic and other tests. For multiple myeloma, various means of assessing therapeutic benefit are described in U.S. Patent No. 7,709,610. All of these various tests can be used to assess therapeutic benefit in the context of patients suffering from multiple myeloma.

### 5.8.2. Voloxicimab Compositions

The voloxicimab compositions described in Section 5.6 are useful for treating a variety of disorders and conditions thought to implicate the α₅β₁integrin, including, for example, a variety of solid tumors. Specific patient populations, formulations, modes of administration and dosage amounts and schedules useful for treating or preventing solid tumors, decreasing angiogenesis and curtailing cancer cell proliferation are described in U.S. Patent No. 7,662,384.
All of these formulations, modes of administration, dosing amounts and schedules, as well as disclosed specific patient populations and combination therapies, are equally suited to the voloxicimab compositions described herein.

The voloxicimab compositions and formulations described herein are administered in amounts that provide therapeutic benefit. Therapeutic benefit includes, but is not limited, to treatment of the underlying disorder. Therapeutic benefit may also include improving or ameliorating symptoms or side effects of a particular disease as assessed using standard diagnostic and other tests. For treatment of tumors, various means of assessing therapeutic benefit are described in U.S. Patent No. 7,662,384. All of these various tests can be used to assess therapeutic benefit in the context of patients suffering from cancer.

### 5.8.3. Enavatuzumab Compositions

The enavatuzumab compositions described in Section 5.6 are useful for treating a variety of disorders and conditions thought to implicate the Tweak Receptor (TweakR), including, for example, a variety of solid tumors. Specific patient populations, formulations, modes of administration and dosage amounts and schedules useful for treating or preventing solid tumors, decreasing angiogenesis and curtailing cancer cell proliferation are described in U.S. Published App. No. 2009/0074762.

All of these formulations, modes of administration, dosing amounts and schedules, as well as disclosed specific patient populations and combination therapies, are equally suited to the enavatuzumab compositions described herein.

The enavatuzumab compositions and formulations described herein are administered in amounts that provide therapeutic benefit. Therapeutic benefit includes, but is not limited, to treatment of the underlying disorder. Therapeutic benefit may also include improving or ameliorating symptoms or side effects of a particular disease as assessed using standard diagnostic and other tests. For treatment of tumors, various means of assessing therapeutic benefit are described in U.S. Published App. No. 2009/0074762. All of these various tests can be used to assess therapeutic benefit in the context of patients suffering from cancer.

### 6. EXAMPLES

Various aspects and features of the inventions described herein are described further by way of the examples, below. Features that are described in association with a particular embodiment (whether in the Summary above or in the examples that follow) can be deviated from without substantially affecting the desirable properties of the methods and compositions of the disclosure, and moreover that different embodiments can be combined and used in various ways together unless they are clearly mutually exclusive. Accordingly, it is to be understood that the examples provided below are intended to be illustrative and not limiting, and should not be construed as limiting the claims that follow.

### 6.1. Production of NS0 Stable Cell Lines

### 6.1.1. Elotuzumab

Mouse myeloma cell line NS0-W (W indicates that the NS0 cells were weaned from their requirement for serum and cholesterol) as described by Hartman et al., 2007, Biotechnol. Bioeng. 96(2):294-306, was maintained in a protein-free basal medium. NS0-W cells were transfected with pHuLuc63 expression plasmid DNA by electroporation. Transfectants that had stably integrated the expression plasmid were selected in the presence of mycophenolic acid in DMEM medium containing 10% fetal bovine serum. Starting from an NS0-W transfectant that produced a high level of HuLuc63, subcloning was performed by limited dilution. A subclone with acceptable productivity and product characteristics was chosen as the final production cell line and designated 192-C17.

### 6.1.2. Daclizumab

Mouse myeloma cell line NS0 was obtained from European Collection of Cell Cultures (ECACC catalog # 85110503, Salisbury, Wiltshire, UK). A vial of these NS0 cells was thawed into DMEM supplemented with 10% FBS. The cells were subsequently cultured in basal medium SFM-3 supplemented with 1 mg/mL BSA. SFM-3 is a 1:1 mixture of DMEM and Ham's F-12 supplemented with 10 mg/L insulin and 10 ug/mL Transferrin. Over a period of approximately 3 months, the NS0 cells were adapted to SFM-3 without supplements, by gradually reducing the amount of FBS present in the culture medium until it was eliminated, and then finally removing BSA in a single step. The resulting host cell line was passaged 15-20 times in SFM-3 and a frozen bank was prepared.

The SFM-3 adapted cells were transfected with pHAT.IgG1.rg.dE by electroporation. Transfectants that had stably integrated the vector were selected in the presence of mycophenolic acid in DMEM medium containing 10% fetal bovine serum. Starting from an NS0 stable transfectant that produced a high level of DAC HYP subcloning was performed by either limited dilution cloning or fluorescence activated cell sorting (FACS). A subclone with acceptable productivity and product characteristics was chosen as the final production cell line and designated 7A11-5H7-14-43.

### 6.1.3. Voloxicimab

The M200-producing cell line, 46-12, was derived from NS0-W cells transfected with the expression plasmid P-200M. Prior to transfection, NS0 cells, received from the ECACC (ECACC No. 85110503), were adapted to serum- and cholesterol-free conditions at PDL and designated NS0-W. NS0-W cells were transfected with P-200M expression plasmid DNA by electroporation. Transfectants that had stably integrated the expression plasmid were selected in the presence of mycophenolic acid in DMEM medium containing 5% fetal bovine serum. Starting from an NS0-W transfectant that produced a high level of M200, subcloning was performed by limited dilution cloning. A subclone with acceptable productivity and product characteristics was chosen as the final production cell line and designated 46-12.

### 6.1.4. PDL241

Mouse myeloma cell line NS0-W (W indicates that the NS0 cells were weaned from their requirement for serum and cholesterol) as described by Hartman et al., 2007, Biotechnol. Bioeng. 96(2):294-306, was maintained in a protein-free basal medium. NS0-W cells were transfected with PDL241 expression plasmid DNA by electroporation. Transfectants that had stably integrated the expression plasmid were selected in the presence of mycophenolic acid in DMEM medium containing 10% fetal bovine serum. Starting from an NS0-W transfectant that produced a high level of PDL241, subcloning was performed by limited dilution cloning. A subclone with acceptable productivity and product characteristics was chosen and designated 26-5C6.

### 6.1.5. Enavatuzumab

Mouse myeloma cell line NS0-W (W indicates that the NS0 cells were weaned from their requirement for serum and cholesterol) as described by Hartman et al., 2007, Biotechnol. Bioeng. 96(2):294-306, was maintained in a protein-free basal medium. NS0-W cells were transfected with pHu19.2.1 expression plasmid DNA by electroporation. Transfectants that had stably integrated the expression plasmid were selected in the presence of mycophenolic acid in DMEM medium containing 10% fetal bovine serum. Starting from an NS0-W transfectant that produced a high level of PDL192, subcloning was performed by limited dilution. A subclone with acceptable productivity and product characteristics was chosen as the final production cell line and designated 299-9.

### 6.2. Production of Antibodies

### 6.2.1. Cell Culture and Recovery

Cells are thawed from a single cell bank vial and expanded in progressively larger volumes within T-flasks, roller bottles, spinner flasks, and bioreactors until the production scale is achieved. Upon completion of the production culture, the cell culture fluid is clarified by centrifugation and depth filtration, and transferred to a harvest hold tank. The production culture duration is approximately 10 days.

Cell culture and recovery can be carried out in a variety of different cell culture facilities using standard equipment, as is known in the art. In another example, cells are thawed from a single cell bank vial and expanded in progressively larger volumes within shaker flasks and bioreactors until the production scale is achieved. Upon completion of the production culture, the cell culture fluid is clarified by centrifugation and depth filtration, and transferred to a harvest hold tank. The production culture duration is approximately 10 days.

### 6.2.1.1. Inoculum Preparation

Production batches are initiated by thawing a single cell bank vial. To generate control media (*i.e.,* without excess glycine), cells are transferred to a T-flask containing a chemically-defined medium, Protein Free Basal Medium-2 (PFBM-2). Custom Powder for making PFBM-2 can be ordered from Invitrogen by requesting Hybridoma-SFM media powder prepared without NaCl, phenol red, transferrin, and insulin, including a quantity of EDTA iron (III) sodium salt that, when reconstituted, yields a concentration of 5 mg/L, and that has quantities of the remaining components adjusted such that, when reconstituted, their concentrations are the same as reconstituted Hybridoma-SFM. Prepared PFBM-2 medium contains the following components: 8 g/L Custom Powder; 2.45 g/L sodium bicarbonate; 3.15 g/L NaCl; and 16.5 g/L D-glucose monohydrate (15 g/L glucose). The concentration of glycine in the PFBM-2 medium is 2 mM.

To produce a glycine medium, the PFBM-2 is first produced as described above. Additional glycine (*e.g.,* 5 mM, 15 nM, or 30 nM) is then added to the PFBM-2 medium. To ensure that the high glycine medium has approximately the same osmolality as the PFBM-2 control medium, osmolality is adjusted by adding an appropriate level of NaCl. For instance, Table 8 shows the amount of NaCl that is added to the control medium and to three high glycine media to achive an osmolality of approximately 300 mOsm/kg.

**Table 8: Concentration of NaCl in control medium and high glycine medium.**

| **Table 8** | | | |
|---|---|---|---|
| Media | NaCl (g/L) | Total Glycine (mM) | Final Osmolality (mOsm/kg) |
| 2L Control | 2.8 | 2 | 295 |
| 2L w/ extra 5mM Gly | 2.8 | 7 | 298 |
| 2L w/ extra 15mM Gly | 2.5 | 17 | 301 |
| 2L w/ extra 30mM Gly | 2.1 | 32 | 300 |

The cells are expanded by serial passage into roller bottles or spinner flasks every two days thereafter. T-flasks, roller bottles, and spinner flasks are placed in an incubator operating under a temperature set point of 37°C under an atmosphere of 7.5% CO₂ for T-flasks and roller bottles and 5% CO₂ for spinner flasks.

The spinner flasks are supplemented with 5% CO₂ either by overlay into the headspace or by sparge into the culture, depending on the cell culture volume, and impeller speed is controlled at constant revolutions per minute (RPM). The target seeding density at all inoculum expansion passages is approximately 2.5 × 10⁵ viable cells/mL.

The cells are expanded by serial passage into shaker flasks every two days thereafter. Shaker flasks are placed in an incubator operating under a temperature set point of 37°C under an atmosphere of 7.5% CO₂.

The shaker flasks are agitated at constant revolutions per minute (RPM) on a shaker platform in the incubators. The target seeding density at all inoculum expansion passages is approximately 2.2 - 2.5 × 10⁵ viable cells/mL.

Approximately 14 days following cell bank thaw, when a sufficient number of viable cells have been produced, the first of several, typically three or four, stainless steel stirred-tank seed bioreactors is inoculated. Prior to use, the seed bioreactors are cleaned-in-place, steamed-in-place, and loaded with the appropriate volume of PFBM-2 culture medium. The pH and dissolved oxygen probes are calibrated prior to the bioreactor being steamed-in-place. The first seed bioreactor is inoculated with a sufficient number of cells to target an initial cell density of 2.0 - 2.5 × 10⁵ viable cells/mL. Sequential transfer to the larger volume (typically, 100L to 300 L and then to the 1,000 L seed bioreactors, or 60L to 235L, 950L, and 3750L seed bioreactors) is performed following approximately two days of growth in each reactor and target initial cell densities of 2.0 - 2.5 × 10⁵ viable cells/mL. Culture pH is maintained by addition of either CO₂ gas or 1 M sodium carbonate (Na₂CO₃) via automatic control. The target operating conditions of the seed and production bioreactors include a temperature set point of 37°C, pH 7.0 and 30% dissolved oxygen (as a percentage of air saturation). The 100L, 300L and 1,000L bioreactors are agitated at 100 rpm, 80 rpm and 70 rpm, respectively. In some instances, the target operating conditions of the seed and production bioreactors include a temperature set point of 37°C, a pH of 7.0 with CO₂ sparge and base addition control and 30% dissolved oxygen (as a percentage of air saturation). The larger volume bioreactors can be agitated at speeds of 100 rpm, 80 rpm, 70 rpm, or 40 rpm.

### 6.2.2. Cell Culture Production Bioreactor

After approximately 2 days in the 1,000 L seed bioreactor, the inoculum is transferred into a stainless steel stirred-tank production bioreactor. The production bioreactor has a working volume of approximately 10,000 L. Prior to use, the bioreactor is cleaned-in-place, steamed-in-place, and loaded with approximately 4,000 L of PFBM-2 control medium or the high glycine media. The pH and dissolved oxygen probes are calibrated prior to the bioreactor being steamed-in-place.

In another example, the inoculum is grown in a 3750L seed bioreactor before transfer to a stainless steel stirred-tank production bioreactor with a working volume of approximately 15,000 L, which is cleaned-in-place, steamed-in-place, and loaded with approximately 4,000-7,000 L of PFBM-2 medium or the high glycine media prior to use.

The target seeding density of the production bioreactor is in the range of 2.0 -2.5 × 10⁵ viable cells/mL. A chemically-defined Protein Free Feed Medium concentrate (PFFM-3) (a chemically-defined concentrated feed medium made by reconstituting PFFM3 subcomponents 1 and 2, L-glutamine, D-glucose, sodium phosphate dibasic heptahydrate, L-tyrosine, folic acid, hydrochloric acid, and sodium hydroxide) is added during culture. PFFM3 contains the components shown in Table 9:

| **Table 9: PFFM3 Medium Components** | |
|---|---|
| **Component** | **Concentration** |
| PFFM3 Subcomponent 1 (amino acids) | 20.4 g/L prepared |
| PFFM3 Subcomponent 2 (vitamins and trace elements) | 4.93 g/L prepared |
| L-Glutamine | 11.0 g/L prepared |
| D-Glucose | 28.0 g/L prepared |
| L-Tyrosine, disodium salt | 1.32 g/L prepared |
| Folic Acid | 0.083 g/L prepared |
| Na₂HPO₄• 7H₂0 | 1.74 g/L prepared |
| Sodium Hydroxide | Varies, pH control |
| Glacial Hydrochloric Acid | Varies, pH control |
| WFI water | |

PFFM3 Subcomponent 1 contains the components shown in Table 10 below:

| **Table 10: PFFM3, Subcomponent 1** | | | |
|---|---|---|---|
| **Medium Components** | **MW (g/mole)** | **Conc. (mg/L)** | **Conc. (mM)** |
| L-Arginine HCl | 211. | 1,900 | 9.00E+00 |
| L-Asparagine Anhydrous | 132.1 | 1,320 | 9.99E+00 |
| L-Aspartic Acid | 133.1 | 119 | 8.94E-01 |
| L-Cysteine HCl•H₂O | 176.0 | 2,030 | 1.15E+01 |
| L-Glutamic Acid | 147.1 | 510 | 3.47E+00 |
| Glycine | 75.1 | 157 | 2.09E+00 |
| L-Histidine HCl•H₂O | 210.0 | 864 | 4.11E+00 |
| L-Isoleucine | 131.2 | 1,440 | 1.10E+01 |
| L-Leucine | 131.2 | 3,130 | 2.39E+01 |
| L-Lysine HCl | 183.0 | 2,160 | 1.18E+01 |
| L-Methionine | 149.2 | 1,260 | 8.45E+00 |
| L-Phenylalanine | 165.2 | 918 | 5.56E+00 |
| L-Proline | 115.1 | 806 | 7.00E+00 |
| L-Serine | 105.1 | 709 | 6.75E+00 |
| L-Threonine | 119.1 | 1,220 | 1.02E+01 |
| L-Tryptophan | 204.2 | 408 | 2.00E+00 |
| L-Valine | 117.1 | 1,450 | 1.24E+01 |

PFFM3 Subcomponent 2 contains components shown in Table 11 below:

| **Table 11: PFFM3, Subcomponent 2** | | | |
|---|---|---|---|
| **Medium Components** | **MW g/mole)** | **Conc. (mg/L)** | **Conc. (mM)** |
| Vitamin B-12 | 1,355.0 | 10.72 | 7.91E-03 |
| Biotin | 244.0 | 0.156 | 6.39E-04 |
| Choline Chloride | 140.0 | 140 | 1.00E+00 |
| I-Inositol | 180.0 | 197 | 1.09E+00 |
| Niacinamide | 122.0 | 31.5 | 2.58E-01 |
| Calcium Pantothenate | 477.0 | 103.1 | 2.16E-01 |
| Pyridoxine Hydrochloride | 206.0 | 0.484 | 2.35E-03 |
| Thiamine Hydrochloride | 337.0 | 99.8 | 2.96E-01 |
| Putrescine 2HCl | 161.1 | 6.66 | 4.13E-02 |
| DL-Lipoic thioctic acid | 206.0 | 4.84 | 2.35E-02 |
| Sodium Pyruvate | 110.0 | 1,716 | 1.56E+01 |
| Ethanolamine HCl | 97.54 | 76.1 | 7.80E-01 |
| β-Mercaptoethanol | 78.13 | 60.9 | 7.80E-01 |
| Linoleic Acid | 280.48 | 0.655 | 2.34E-03 |
| Pluronic F-68 | 8,350.0 | 780 | 9.34E-02 |
| Potassium Chloride | 74.55 | 432 | 5.79E+00 |
| Riboflavin | 376.0 | 3.42 | 9.09E-03 |
| Magnesium Chloride Anhyd. | 95.21 | 446 | 4.69E+00 |
| Magnesium Sulfate Anhyd. | 120.4 | 762 | 6.33E+00 |
| Sodium Selenite | 172.9 | 0.140 | 8.12E-04 |
| Cupric Sulfate•5H₂O | 249.7 | 0.1069 | 4.28E-04 |
| Ferrous Sulfate•7H₂O | 278.0 | 6.51 | 2.34E-02 |
| Potassium Nitrate | 101.1 | 0.593 | 5.86E-03 |
| Zinc Sulfate•7H₂O | 287.5 | 15.0 | 5.23E-02 |
| Manganese Sulfate, Monohydrate | 169.01 | 0.00264 | 1.56E-05 |
| Nickelous Chloride, 6-Hydrate | 237.7 | 0.00186 | 7.81E-06 |
| Stannous Chloride 2H₂O | 225.63 | 0.001130 | 5.01E-06 |
| Ammonium Molybdate 4H₂O | 1,235.86 | 0.00193 | 1.57E-06 |
| Ammonium meta-Vanadate | 116.98 | 0.00913 | 7.80E-05 |
| Sodium meta-Silicate 9H₂O | 284.2 | 2.22 | 7.79E-03 |
| EDTA, Iron(III), Sodium Salt | 367.05 | 31.2 | 8.50E-02 |

The timing and amount of addition of PFFM-3 to the culture occurs as shown in Table 12 below:

| **Table 12: Exemplary DAC HYP Bioreactor Feed Schedule** | |
|---|---|
| **Day** | **PFFM-3 Amount (% of initial mass)** |
| 0 | 0 |
| 1 | 0 |
| 2 | 4-4.14 |
| 3 | 7.8-8.08 |
| 4 | 7.8-8.08 |
| 5 | 7.8-8.08 |
| 6 | 11-11.38 |
| 7 | 13-13.46 |
| 8 | 15-15.52 |
| 9 | 15-15.52 |
| 10 | 0 |

Culture pH is maintained at approximately pH 7.0, preferably between pH 7.0 and pH 7.1, by automatic control of CO₂ gas and 1 M sodium carbonate (Na₂CO₃) addition. Dissolved oxygen content is allowed to drop to approximately 30% of air saturation. An oxygen/air mixture is sparged into the culture to achieve a constant total gas flow rate and dissolved oxygen is controlled by adjusting the ratio of air to oxygen gases as needed and by increasing agitation speed after reaching a maximum oxygen to air ratio. In another example, agitation is adjusted to maintain a constant power/volume ratio. A simethicone-based antifoam emulsion is added to the bioreactor on an as needed basis based on foam level. Samples are taken periodically to test for cell density, cell viability, product concentration, glucose and lactate concentrations, dissolved O₂, dissolved CO₂, pH, and osmolality. The bioreactor culture is harvested approximately 10 days post-inoculation. Prior to harvest, the bioreactor contents are sampled as unprocessed bulk.

It will be understood that the feed conditions and the feeding schedule can be adjusted for the production of each antibody to optimize titer. The osmolality of the culture medium may be adjusted as needed ti obtain optimal titer. Additionally, glucose and glutamine levels may be adjusted during the production phase to maintain optimal titers.

### 6.2.3. Harvest and Cell Removal

Just prior to harvest, the production bioreactor is first chilled to < 15°C, then adjusted to a pH of 5.0 ± 0.1 using 0.5 M or 1 M or 2 M citric acid, and held for a period of approximately 30 - 90 or 45 - 60 minutes to flocculate the cells and cell debris prior to transfer to the harvest vessel. The pH-adjusted harvest is then clarified by continuous centrifugation operated under predefined parameters for bowl speed and flow rate as defined in batch record documentation.

The centrate is filtered through a depth filter followed by a 0.22 µm membrane filter and collected in a pre-sterilized tank. The cell-free harvest is adjusted to an approximate pH of 6.4 using a 1-2 M Tris solution and stored at 2-8°C for further processing. In some instances, this pH adjustment occurs within 12 hours of the original bioreactor pH adjustment to pH 5.0.

### 6.2.4. Antibody Purification

Harvest materials from pilot scale runs (50 liters and above) were purified based on three chromatography techniques (MabSelect Protein A affinity chromatography, Q-Sepharose anion exchange chromatography, and CM-Sepharose cation exchange chromatography) in combination with a low pH viral inactivation step, a viral filtration step, an ultrafiltration/diafiltration step, and a formulation step. Materials from 2 liter harvest were processed through Protein A column followed by Size-Exclusion chromatography.

### 6.3. Determination of Glycosylation Profile

### 6.3.1. Materials and Methods

Purified antibodies from different culture conditions (control condition with low glycine vs. experimental condition with high glycine) were analytically characterized. Peptide mapping was used to quantify different glycoforms in the antibody products. Antibody was first reduced with dithiothreitol, alkylated with iodoacetic acid, and then digested with trypsin, and finally analyzed by RP-HPLC with MS detection. The peak areas of each glycoform were obtained and the relative percentage of each glycoform was then calculated from the total peak areas.

### 6.3.2. Results

The relative percentages of each glycoform present in the five different antibodies produced from NS0 cells cultured in either control basal medium containing 2 mM of glycine or basal medium supplemented with 15 mM (with a total concentration of 17 mM) are displayed in Table 13. The percentage increase of the two non-fucosylated glycoforms for five different antibodies, G0 -GlcNAc-Fucose glycoform and Man5 glycoform, formed in the medium supplemented with glycine relative to the control medium are shown in **FIGURE 2** and **FIGURE 3****.** The combined total percentage increase of non-fucosylated antibody relative to the control is shown in **FIGURE 4****.** The data indicate that antibodies from cells cultured under high concentration of glycine exhibit higher levels of non-fucosylated glycoforms than the same antibodies from the control process with a low concentration of glycine. With respect to the G0 -GlcNAc-Fucose glycoform, four of the five antibodies from cells cultured in the higher glycine concentration culture medium exhibit 150% to almost 300% of this glycoform relative to the control. With respect to the Man5 glycoform, the range for four of the five antibodies is 150% to >200% increase of this glycoform relative to the control. Looking collectively at the total amount of non-fucosylated antibody, all five of the antibodies exhibit a 1.5 -2.5 times increase when cells are cultured in the higher glycine concentration media.

**Table 13: Glycosylation profile of four antibodies produced by methods in accordance with the present disclosure**

| **TABLE 13** | | | | | | |
|---|---|---|---|---|---|---|
| **Elotuzumab** | **G0-GlcNAc-Fucose** | **Man5** | **Total Non-fucosylated Ab** | **G0-GlcNAc** | **G0** | **G1** |
| **1kL control** | **1.6** | **2**.**0** | **3.6** | *13.3* | *68.9* | *14.2* |
| 100L with extra glycine | 2.7 | 4,4 | 7.1 | *13.3* | *70.2* | *9.4* |
| **PDL241** | | | | | | |
| **2L control** | **4.9** | **7.4** | **12.3** | *20.6* | *58.0* | *9.1* |
| 2L with extra glycine | 6.0 | 11.8 | 17.7 | *20.7* | *56.6* | *5.0* |
| **PDL192** | | | | | | |
| **1kL control** | **1.8** | **2.1** | **3.9** | *9.7* | *76*.*7* | *9.7* |
| 50.L with extra glycine | 4.2 | 4.4 | 8.6 | *11.6* | *75.2* | *4.5* |
| **Daclizumab** | | | | | | |
| **2L control** | **1.4** | **1.0** | **2.4** | *7.7* | *72.5* | *17.4* |
| 2L with extra glycine | 4 | 1.9 | 5.9 | *14.6* | *69.3* | *10.2* |
| **M200** | | | | | | |
| **2L control** | **0.4** | **0.4** | **0.8** | *4.3* | *66.1* | *28.8* |
| 2L with extra glycine | 0.7 | 0.5 | 1.2 | *5.7* | *80.2* | *12.9* |

In the dose response study, the percentages of each glycoform present in antibody PDL192 (enavatuzumab) produced from cells cultured in either control basal medium containing 2 mM glycine or basal medium supplemented with different concentrations of glycine (7, 17, and 32 mM) are displayed in Table 14. The percentage increase of the two non-fucosylated glycoforms of PDL192, G0 -GlcNAc-Fucose glycoform and Man5 glycoform, formed in the medium supplemented with glycine relative to the control medium are shown in **FIGURE 5****.** The data indicate that PDL192 produced under conditions with extra glycine exhibit a higher level of non-fucosylated glycoforms than the antibody produced from the control process. More specifically for the non-fucosylated G0-GlcNAc-fucose glycoform, the amount of the glycoform increases with increased concentration of glycine in the culture medium in a dose-dependent manner. The percentage of increase ranges from 120% to >160% relative to the control as glycine concentration increased from 7 to 32 mM.

**Table 14: Glycosylation profile and CD16a binding of PDL192 produced in culture media with excess glycine**

| **TABLE 14** | | | | | | | |
|---|---|---|---|---|---|---|---|
| **PDL192** | **G0-GlcNAc-Fucose** | **Man5** | **Total Non-fucosylated Ab** | **G0-GlcNAc** | **G0** | **G1** | **CD16a Binding relative to Reference** |
| **2L control** | **2.1** | **5.2** | **7.3** | *12.3* | *70.6* | *9.8* | 130 |
| **2L with extra 5 mM glycine** | **2.6** | **6.3** | **8.9** | *15.4* | *71.3* | *4.4* | 146 |
| **2L with extra 15 mM glycine** | **3.3** | **7.7** | **11.0** | *13.5* | *70.5* | *4.9* | 151 |
| **2L with extra 30 mM glycine** | **3.5** | **6.3** | **9.8** | *14.6* | *71.5* | *4.0* | 144 |

### 6.4. CD16a Binding Assay

### 6.4.1. Materials and Methods

A CD16a binding assay was performed using AlphaScreen (PerkinElmer) method to assess the binding potency of the antibody Fc region of four different antibodies to human CD16a receptor. The antibody solution was first mixed with a recombinant human CD16a solution and assay buffer, and then Alphascreen donor beads and antibody acceptor beads were added. The mixture was incubated in a light protected location for 4 hours at room temperature and the fluorescent signal was detected using a plate reader with laser excitation at 680 nm and light emission 520-620nm. The binding potency relative to the antibody reference cultured in a basal medium without excess glycine was reported.

### 6.4.2. Results

The results from the CD16a potency assays for four different IgG1antibodies (elotuzumab, daclizumab, PDL192 and PDL241) are shown in Table 15. All four IgG1 antibodies from cells cultured under the high concentration of glycine consistently showed more than a 40% increase in binding affinity to the human CD16a receptor, and in the most extreme case exhibited a twofold increase in terms of binding potency relative to the control antibodies produced in basal media without excess glycine (*i.e.,* culture media 2 mM glycine). **FIGURE 6** shows the relative binding potency to the CD16a receptor for the four IgG1 antibodies. The amount is expressed as a percentage relative to the CD16a binding affinities of the same antibodies produced by NS0 cells cultured in control basal medium without additional glycine supplementation.

**Table 15: CD16a Binding of four antibodies produced by methods in accordance with the present disclosure**

| **TABLE 15** | |
|---|---|
| **Elotuzumab** | **CD16a Binding Relative to Reference** |
| **1kL control** | **100%** |
| 100L with extra glycine | 128% |
| 1kL with extra glycine | 146% |
| | |
| **PDL241** | |
| **2L control** | 147% |
| 2L with extra glycine | 277% |
| **250L control** | **100%** |
| 100L with extra glycine | 137% |
| | |
| **PDL192** | |
| 1kL control (GMP) | 65% |
| **1kL control** | **96%** |
| 100L control | 110% |
| 58L with extra glycine | **136%** |
| | |
| **Daclizumab** | |
| **2L control** | **69%** |
| 2L with extra glycine | 137% |

The CD16a potency results from dose response study on PDL192 are shown in Table 14 and **FIGURE 7****.** PDL192 from cells cultured under conditions supplemented with extra glycine of 5, 15 and 30 mM showed higher CD16a binding affinity than the antibody from the control process, although a dose-dependent increase in CD16a binding was not observed.

### 6.5. Antibody-Dependent Cellular Cytotoxicity (ADCC)

### 6.5.1. Materials and Methods

ADCC activity of the expressed antibody was analyzed *in vitro.* Target cells were first labeled with ⁵¹Cr and effector cells (human peripheral blood mononuclear cells, PBMC) were prepared from whole blood. The cells and antibody solution were then incubated at 37°C for 4 hours. The radioactivity in the supernatants was measured for experimental release (E), spontaneous release (S, release from target without effector cells and antibody), and total lysate (T, release from target cells treated with detergent 1% Triton X-100). The percent cytotoxicity was calculated as [(E-S)/(T-S)]×100.

### 6.5.2. Results

The results from the ADCC assays of four different antibodies (elotuzumab, PDL241, PDL192 and daclizumab) are shown in **FIGURES 8A-C****,** **9A-B****,** **10A-B** and **11A-B****,** with each assay performed on PBMCs from two or three different donors. In all four figures, antibodies from cells cultured under the higher concentration of glycine showed increased ADCC activity compared to the same antibodies from cells cultured under the control process. The results indicate that ADCC activity is consistent with CD16a binding potency, and both assay results correlate with the glycosylation patterns of the antibodies.

## Claims

1. A method of producing an IgG1 antibody, comprising:
culturing NSO cells engineered to secrete and express the IgG1 antibody in a cell culture medium comprising glycine at a concentration between 5 mM and 30 mM under conditions suitable for expression and secretion of the IgG1 antibody, thereby producing the IgG1 antibody.

2. The method of claim 1, wherein the concentration of glycine is in the range of from 10 mM to 25 mM, or 15 mM to 20 mM, or 16 mM to 18 mM.

3. The method of any one of claims 1-2, wherein the medium is a protein- free medium.

4. The method of any one of claim 1-3, further comprising recovering the IgG1 antibody.

5. The method of claim 4, wherein recovering the IgG1 antibody comprises the step of separating the NS0 cells from the culture medium, and optionally purifying the IgG1 antibody.

6. The method of any one of claims 1-5, wherein the NSO cells are seeded to a density of 1.5 X 10⁵ cells/mL to 2.5 X 10⁵ cells/mL, prior to said culturing.

7. The method of any one of claims 1-6, wherein the IgG1 antibody comprising a VH region of the amino acid sequence of SEQ ID NO: and a VL region of the amino acid sequence of SEQ ID NO:2, optionally wherein the IgG1 antibody has a full heavy chain amino acid sequence of SEQ ID NO:3 and a full light chain amino acid sequence of SEQ ID NO:4.

8. The method of any one of claims 1-6, wherein the IgG1 antibody comprising a VH region of the amino acid sequence of SEQ ID NO:5 and a VL region of the amino acid sequence of SEQ ID NO:6.

9. The method of claim 8, wherein the IgG1 antibody has a full heavy chain amino acid sequence of SEQ ID NO:7 and a full light chain amino acid sequence of SEQ ID NO:8.

10. The method of any one of claims 1-6, wherein the IgG1 antibody comprising a VH region of the amino acid sequence of SEQ ID NO:9 and a VL region of the amino acid sequence of SEQ ID NO:10.

11. The method of claim 10, wherein the IgG1 antibody has a full heavy chain amino acid sequence of SEQ ID NO:11 and a full light chain amino acid sequence of SEQ ID NO: 12.

12. The method of any one of claims 1-6, wherein the IgG1 antibody comprising a VH region of the amino acid sequence of SEQ ID NO: 13 and a VL region of the amino acid sequence of SEQ ID NO:14.

13. The method of claim 12, wherein the IgG1 antibody has a full heavy chain amino acid sequence of SEQ ID NO: 15 and a full light chain amino acid sequence of SEQ ID NO: 16.

14. The method of any one of claims 1-6, wherein the IgG1 antibody comprising a VH region of the amino acid sequence of SEQ ID NO: 17 and a VL region of the amino acid sequence of SEQ ID NO:18.

15. The method of claim 14, wherein the IgG1 antibody has a full heavy chain amino acid sequence of SEQ ID NO: 19 and a full light chain amino acid sequence of SEQ ID NO:20.

## Patentansprüche

1. Verfahren des Produzierens eines IgG1-Antikörpers, umfassend:
in Kultur nehmen von NSO-Zellen, die engineert sind, den IgG1-Antikörper in einem Zellkulturmedium abzusondern und zu exprimieren, das Glycin bei einer Konzentration zwischen 5 mM und 30 mM umfasst, unter geeigneten Bedingungen zur Expression und Absonderung des IgG1-Antikörpers, wodurch der IgG1-Antikörper produziert wird.

2. Verfahren nach Anspruch 1, wobei die Konzentration von Glycin im Bereich von 10 mM bis 25 mM oder 15 mM bis 20 mM oder 16 mM bis 18 mM liegt.

3. Verfahren nach einem der Ansprüche 1 bis 2, wobei das Medium ein proteinfreies Medium ist.

4. Verfahren nach einem der Ansprüche 1 bis 3, ferner umfassend die Rückgewinnung des IgG1-Antikörpers.

5. Verfahren nach Anspruch 4, wobei die Rückgewinnung des IgG1-Antikörpers den Schritt des Trennens der NS0-Zellen vom Kulturmedium umfasst und optional das Reinigen des IgG1-Antikörpers.

6. Verfahren nach einem der Ansprüche 1 bis 5, wobei die NSO-Zellen zu einer Dichte von 1,5 X 10⁵ Zellen/mL bis 2,5 X 10⁵ Zellen/mL vor dem in Kultur nehmen gesät werden.

7. Verfahren nach einem der Ansprüche 1 bis 6, wobei der IgG1-Antikörper eine VH-Region der Aminosäuresequenz der SEQ ID NR: 1 und eine VL-Region der Aminosäuresequenz der SEQ ID NR:2 umfasst, wobei der IgG1-Antikörper optional eine vollständige Schwerketten-Aminosäuresequenz der SEQ ID NR:3 und eine vollständige Leichtketten-Aminosäuresequenz der SEQ ID NR:4 aufweist.

8. Verfahren nach einem der Ansprüche 1 bis 6, wobei der IgG1-Antikörper eine VH-Region der Aminosäuresequenz der SEQ ID NR:5 und eine VL-Region der Aminosäuresequenz der SEQ ID NR:6 umfasst.

9. Verfahren nach Anspruch 8, wobei der IgG1-Antikörper eine vollständige Schwerketten-Aminosäuresequenz der SEQ ID NR:7 und eine vollständige Leichtketten-Aminosäuresequenz der SEQ ID NR:8 aufweist.

10. Verfahren nach einem der Ansprüche 1 bis 6, wobei der IgG1-Antikörper eine VH-Region der Aminosäuresequenz der SEQ ID NR:9 und eine VL-Region der Aminosäuresequenz der SEQ ID NR:10 umfasst.

11. Verfahren nach Anspruch 10, wobei der IgG1-Antikörper eine vollständige Schwerketten-Aminosäuresequenz der SEQ ID NR: 11 und eine vollständige Leichtketten-Aminosäuresequenz der SEQ ID NR: 12 aufweist.

12. Verfahren nach einem der Ansprüche 1 bis 6, wobei der IgG1-Antikörper eine VH-Region der Aminosäuresequenz der SEQ ID NR:13 und eine VL-Region der Aminosäuresequenz der SEQ ID NR: 14 umfasst.

13. Verfahren nach Anspruch 12, wobei der IgG1-Antikörper eine vollständige Schwerketten-Aminosäuresequenz der SEQ ID NR:15 und eine vollständige Leichtketten-Aminosäuresequenz der SEQ ID NR: 16 aufweist.

14. Verfahren nach einem der Ansprüche 1 bis 6, wobei der IgG1-Antikörper eine VH-Region der Aminosäuresequenz der SEQ ID NR: 17 und eine VL-Region der Aminosäuresequenz der SEQ ID NR:18 umfasst.

15. Verfahren nach Anspruch 14, wobei der IgG1-Antikörper eine vollständige Schwerketten-Aminosäuresequenz der SEQ ID NR: 19 und eine vollständige Leichtketten-Aminosäuresequenz der SEQ ID NR:20 aufweist.

## Revendications

1. Méthode de production d'un anticorps IgG1, comprenant :
la mise en culture de cellules NSO génétiquement modifiées pour sécréter et exprimer l'anticorps IgG1 dans un milieu de culture cellulaire comprenant de la glycine à une concentration allant de 5 mM à 30 mM dans des conditions favorables à l'expression et la sécrétion de l'anticorps IgG1, pour ainsi produire l'anticorps IgG1.

2. Méthode selon la revendication 1, dans laquelle la concentration en glycine est comprise dans une plage allant de 10 mM à 25 mM, ou de 15 mM à 20 mM, ou de 16 mM à 18 mM.

3. Méthode selon l'une quelconque des revendications 1 et 2, dans laquelle le milieu est un milieu exempt de protéines.

4. Méthode selon l'une quelconque des revendications 1 à 3, comprenant en outre la récupération de l'anticorps IgG1.

5. Méthode selon la revendication 4, dans laquelle la récupération de l'anticorps IgG1 comprend l'étape consistant à séparer les cellules NSO du milieu de culture, et éventuellement à purifier l'anticorps IgG1.

6. Méthode selon l'une quelconque des revendications 1 à 5, dans laquelle les cellules NSO sont ensemencées à une densité allant de 1,5 x 10⁵ cellules/mL à 2,5 x 10⁵ cellules/mL, préalablement à ladite mise en culture.

7. Méthode selon l'une quelconque des revendications 1 à 6, dans laquelle l'anticorps IgG1 comprend une région VH de la séquence d'acides aminés de la SEQ ID N° : 1 et une région VL de la séquence d'acides aminés de la SEQ ID N° : 2, l'anticorps IgG1 présentant éventuellement une séquence d'acides aminés à chaîne lourde complète de la SEQ ID N° : 3 et une séquence d'acides aminés à chaîne légère complète de la SEQ ID N° : 4.

8. Méthode selon l'une quelconque des revendications 1 à 6, dans laquelle l'anticorps IgG1 comprend une région VH de la séquence d'acides aminés de la SEQ ID N° : 5 et une région VL de la séquence d'acides aminés de la SEQ ID N° : 6.

9. Méthode selon la revendication 8, dans laquelle l'anticorps IgG1 présente une séquence d'acides aminés à chaîne lourde complète de la SEQ ID N° : 7 et une séquence d'acides aminés à chaîne légère complète de la SEQ ID N° : 8.

10. Méthode selon l'une quelconque des revendications 1 à 6, dans laquelle l'anticorps IgG1 comprend une région VH de la séquence d'acides aminés de la SEQ ID N° : 9 et une région VL de la séquence d'acides aminés de la SEQ ID N° : 10.

11. Méthode selon la revendication 10, dans laquelle l'anticorps IgG1 présente une séquence d'acides aminés à chaîne lourde complète de la SEQ ID N° : 11 et une séquence d'acides aminés à chaîne légère complète de la SEQ ID N° : 12.

12. Méthode selon l'une quelconque des revendications 1 à 6, dans laquelle l'anticorps IgG1 comprend une région VH de la séquence d'acides aminés de la SEQ ID N° : 13 et une région VL de la séquence d'acides aminés de la SEQ ID N° : 14.

13. Méthode selon la revendication 12, dans laquelle l'anticorps IgG1 présente une séquence d'acides aminés à chaîne lourde complète de la SEQ ID N° : 15 et une séquence d'acides aminés à chaîne légère complète de la SEQ ID N° : 16.

14. Méthode selon l'une quelconque des revendications 1 à 6, dans laquelle l'anticorps IgG1 comprend une région VH de la séquence d'acides aminés de la SEQ ID N° : 17 et une région VL de la séquence d'acides aminés de la SEQ ID N° : 18.

15. Méthode selon la revendication 14, dans laquelle l'anticorps IgG1 présente une séquence d'acides aminés à chaîne lourde complète de la SEQ ID N° : 19 et une séquence d'acides aminés à chaîne légère complète de la SEQ ID N° : 20.


### SEQUENCE LISTING

<110> ABBVIE BIOTHERAPEUTICS INC.
<120> COMPOSITIONS AND METHODS FOR PRODUCING GLYCOPROTEINS
<130> 381493-735WO (118134)
<140>
   <141>
<150> 61/708,554
   <151> 2012-10-01
<160> 21
<170> PatentIn version 3.5
<210> 1
   <211> 119
   <212> PRT
   <213> Artificial Sequence
<220>
   <221> source
   <223> /note="Description of Artificial Sequence: Synthetic polypeptide"
<400> 1
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asp Phe Ser Arg Tyr
               20                  25                  30
   Trp Met Ser Trp Val Arg Gln Ala Pro Gly Lys Gly Leu Glu Trp Ile
           35                  40                  45
   Gly Glu Ile Asn Pro Asp Ser Ser Thr Ile Asn Tyr Ala Pro Ser Leu
       50                  55                  60
   Lys Asp Lys Phe Ile Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Pro Asp Gly Asn Tyr Trp Tyr Phe Asp Val Trp Gly Gln Gly
               100                 105                 110
   Thr Leu Val Thr Val Ser Ser
           115
<210> 2
   <211> 107
   <212> PRT
   <213> Artificial Sequence
<220>
   <221> source
   <223> /note="Description of Artificial Sequence: Synthetic polypeptide"
<400> 2
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Val Gly Ile Ala
               20                  25                  30
   Val Ala Trp Tyr Gln Gln Lys Pro Gly Lys Val Pro Lys Leu Leu Ile
           35                  40                  45
   Tyr Trp Ala Ser Thr Arg His Thr Gly Val Pro Asp Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Gln Tyr Ser Ser Tyr Pro Tyr
                   85                  90                  95
   Thr Phe Gly Gln Gly Thr Lys Val Glu Ile Lys
               100                 105
<210> 3
   <211> 467
   <212> PRT
   <213> Artificial Sequence
<220>
   <221> source
   <223> /note="Description of Artificial Sequence: Synthetic polypeptide"
<400> 3
   Met Asp Phe Gly Leu Ile Phe Phe Ile Val Ala Leu Leu Lys Gly Val
   1               5                   10                  15
   Gln Cys Glu Val Gln Leu Val Glu Ser Gly Gly Gly Leu Val Gln Pro
               20                  25                  30
   Gly Gly Ser Leu Arg Leu Ser Cys Ala Ala Ser Gly Phe Asp Phe Ser
           35                  40                  45
   Arg Tyr Trp Met Ser Trp Val Arg Gln Ala Pro Gly Lys Gly Leu Glu
       50                  55                  60
   Trp Ile Gly Glu Ile Asn Pro Asp Ser Ser Thr Ile Asn Tyr Ala Pro
   65                  70                  75                  80
   Ser Leu Lys Asp Lys Phe Ile Ile Ser Arg Asp Asn Ala Lys Asn Ser
                   85                  90                  95
   Leu Tyr Leu Gln Met Asn Ser Leu Arg Ala Glu Asp Thr Ala Val Tyr
               100                 105                 110
   Tyr Cys Ala Arg Pro Asp Gly Asn Tyr Trp Tyr Phe Asp Val Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Ser Ser Lys Ser Thr Ser Gly Gly Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Ser Leu Gly Thr Gln Thr Tyr Ile Cys Asn Val Asn His
       210                 215                 220
   Lys Pro Ser Asn Thr Lys Val Asp Lys Lys Val Glu Pro Lys Ser Cys
   225                 230                 235                 240
   Asp Lys Thr His Thr Cys Pro Pro Cys Pro Ala Pro Glu Leu Leu Gly
                   245                 250                 255
   Gly Pro Ser Val Phe Leu Phe Pro Pro Lys Pro Lys Asp Thr Leu Met
               260                 265                 270
   Ile Ser Arg Thr Pro Glu Val Thr Cys Val Val Val Asp Val Ser His
           275                 280                 285
   Glu Asp Pro Glu Val Lys Phe Asn Trp Tyr Val Asp Gly Val Glu Val
       290                 295                 300
   His Asn Ala Lys Thr Lys Pro Arg Glu Glu Gln Tyr Asn Ser Thr Tyr
   305                 310                 315                 320
   Arg Val Val Ser Val Leu Thr Val Leu His Gln Asp Trp Leu Asn Gly
                   325                 330                 335
   Lys Glu Tyr Lys Cys Lys Val Ser Asn Lys Ala Leu Pro Ala Pro Ile
               340                 345                 350
   Glu Lys Thr Ile Ser Lys Ala Lys Gly Gln Pro Arg Glu Pro Gln Val
           355                 360                 365
   Tyr Thr Leu Pro Pro Ser Arg Asp Glu Leu Thr Lys Asn Gln Val Ser
       370                 375                 380
   Leu Thr Cys Leu Val Lys Gly Phe Tyr Pro Ser Asp Ile Ala Val Glu
   385                 390                 395                 400
   Trp Glu Ser Asn Gly Gln Pro Glu Asn Asn Tyr Lys Thr Thr Pro Pro
                   405                 410                 415
   Val Leu Asp Ser Asp Gly Ser Phe Phe Leu Tyr Ser Lys Leu Thr Val
               420                 425                 430
   Asp Lys Ser Arg Trp Gln Gln Gly Asn Val Phe Ser Cys Ser Val Met
           435                 440                 445
   His Glu Ala Leu His Asn His Tyr Thr Gln Lys Ser Leu Ser Leu Ser
       450                 455                 460
   Pro Gly Lys
   465
<210> 4
   <211> 234
   <212> PRT
   <213> Artificial Sequence
<220>
   <221> source
   <223> /note="Description of Artificial Sequence: Synthetic polypeptide"
<400> 4
   Met Glu Thr His Ser Gln Val Phe Val Tyr Met Leu Leu Trp Leu Ser
   1               5                   10                  15
   Gly Val Glu Gly Asp Ile Gln Met Thr Gln Ser Pro Ser Ser Leu Ser
               20                  25                  30
   Ala Ser Val Gly Asp Arg Val Thr Ile Thr Cys Lys Ala Ser Gln Asp
           35                  40                  45
   Val Gly Ile Ala Val Ala Trp Tyr Gln Gln Lys Pro Gly Lys Val Pro
   50 55 60
   Lys Leu Leu Ile Tyr Trp Ala Ser Thr Arg His Thr Gly Val Pro Asp
   65 70 75 80
   Arg Phe Ser Gly Ser Gly Ser Gly Thr Asp Phe Thr Leu Thr Ile Ser
   85 90 95
   Ser Leu Gln Pro Glu Asp Val Ala Thr Tyr Tyr Cys Gln Gln Tyr Ser
   100 105 110
   Ser Tyr Pro Tyr Thr Phe Gly Gln Gly Thr Lys Val Glu Ile Lys Arg
   115 120 125
   Thr Val Ala Ala Pro Ser Val Phe Ile Phe Pro Pro Ser Asp Glu Gln
   130 135 140
   Leu Lys Ser Gly Thr Ala Ser Val Val Cys Leu Leu Asn Asn Phe Tyr
   145 150 155 160
   Pro Arg Glu Ala Lys Val Gln Trp Lys Val Asp Asn Ala Leu Gln Ser
   165 170 175
   Gly Asn Ser Gln Glu Ser Val Thr Glu Gln Asp Ser Lys Asp Ser Thr
   180 185 190
   Tyr Ser Leu Ser Ser Thr Leu Thr Leu Ser Lys Ala Asp Tyr Glu Lys
   195 200 205
   His Lys Val Tyr Ala Cys Glu Val Thr His Gln Gly Leu Ser Ser Pro
   210 215 220
   Val Thr Lys Ser Phe Asn Arg Gly Glu Cys
   225 230
<210> 5
   <211> 116
   <212> PRT
   <213> Artificial Sequence
<220>
   <221> source
   <223> /note="Description of Artificial Sequence: Synthetic polypeptide"
<400> 5
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Thr Phe Thr Ser Tyr
               20                  25                  30
   Arg Met His Trp Val Arg Gln Ala Pro Gly Gln Gly Leu Glu Trp Ile
           35                  40                  45
   Gly Tyr Ile Asn Pro Ser Thr Gly Tyr Thr Glu Tyr Asn Gln Lys Phe
       50                  55                  60
   Lys Asp Lys Ala Thr Ile Thr Ala Asp Glu Ser Thr Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Gly Val Phe Asp Tyr Trp Gly Gln Gly Thr Thr Leu
               100                 105                 110
   Thr Val Ser Ser
           115
<210> 6
   <211> 107
   <212> PRT
   <213> Artificial Sequence
<220>
   <221> source
   <223> /note="Description of Artificial Sequence: Synthetic polypeptide"
<400> 6
   Asp Ile Gln Met Thr Gln Ser Pro Ser Thr Leu Ser Ala Ser Val Gly
   1               5                   10                  15
   Asp Arg Val Thr Ile Thr Cys Ser Ala Ser Ser Ser Ile Ser Tyr Met
               20                  25                  30
   His Trp Tyr Gln Gln Lys Pro Gly Lys Ala Pro Lys Leu Leu Ile Tyr
           35                  40                  45
   Thr Thr Ser Asn Leu Ala Ser Gly Val Pro Ala Arg Phe Ser Gly Ser
       50                  55                  60
   Gly Ser Gly Thr Glu Phe Thr Leu Thr Ile Ser Ser Leu Gln Pro Asp
   65                  70                  75                  80
   Asp Phe Ala Thr Tyr Tyr Cys His Gln Arg Ser Thr Tyr Pro Leu Thr
                   85                  90                  95
   Phe Gly Ser Gly Thr Lys Val Glu Val Lys Arg
               100                 105
<210> 7
   <211> 442
   <212> PRT
   <213> Artificial Sequence
<220>
   <221> source
   <223> /note="Description of Artificial Sequence: Synthetic polypeptide"
<400> 7
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Thr Phe Thr Ser Tyr
               20                  25                  30
   Arg Met His Trp Val Arg Gln Ala Pro Gly Gln Gly Leu Glu Trp Ile
           35                  40                  45
   Gly Tyr Ile Asn Pro Ser Thr Gly Tyr Thr Glu Tyr Asn Gln Lys Phe
       50                  55                  60
   Lys Asp Lys Ala Thr Ile Thr Ala Asp Glu Ser Thr Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Gly Val Phe Asp Tyr Trp Gly Gln Gly Thr Thr Leu
               100                 105                 110
   Thr Val Ser Ser Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Ala Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Tyr Thr Leu Pro Pro Ser Arg Asp Glu
               340                 345                 350
   Leu Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
   435 440
<210> 8
   <211> 210
   <212> PRT
   <213> Artificial Sequence
<220>
   <221> source
   <223> /note="Description of Artificial Sequence: Synthetic polypeptide"
<400> 8
   Asp Ile Gln Met Thr Gln Ser Pro Ser Thr Leu Ser Ala Ser Val Gly
   1               5                   10                  15
   Asp Arg Val Thr Ile Thr Cys Ser Ala Ser Ser Ser Ile Ser Tyr Met
               20                  25                  30
   His Trp Tyr Gln Gln Lys Pro Gly Lys Ala Pro Lys Leu Leu Ile Tyr
           35                  40                  45
   Thr Thr Ser Asn Leu Ala Ser Gly Val Pro Ala Arg Phe Ser Gly Ser
       50                  55                  60
   Gly Ser Gly Thr Glu Phe Thr Leu Thr Ile Ser Ser Leu Gln Pro Asp
   65                  70                  75                  80
   Asp Phe Ala Thr Tyr Tyr Cys His Gln Arg Ser Thr Tyr Pro Leu Thr
                   85                  90                  95
   Phe Gly Ser Gly Thr Lys Val Glu Val Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
                 195                 200                 205
   Asn Arg
       210
<210> 9
   <211> 124
   <212> PRT
   <213> Artificial Sequence
<220>
   <221> source
   <223> /note="Description of Artificial Sequence: Synthetic polypeptide"
<400> 9
   Gln Val Gln Leu Lys Glu Ser Gly Pro Gly Leu Val Ala Pro Ser Gln
   1               5                   10                  15
   Ser Leu Ser Ile Thr Cys Thr Ile Ser Gly Phe Ser Leu Thr Asp Tyr
               20                  25                  30
   Gly Val His Trp Val Arg Gln Pro Pro Gly Lys Gly Leu Glu Trp Leu
           35                  40                  45
   Val Val Ile Trp Ser Asp Gly Ser Ser Thr Tyr Asn Ser Ala Leu Lys
       50                  55                  60
   Ser Arg Met Thr Ile Arg Lys Asp Asn Ser Lys Ser Gln Val Phe Leu
   65                  70                  75                  80
   Ile Met Asn Ser Leu Gln Thr Asp Asp Ser Ala Met Tyr Tyr Cys Ala
                   85                  90                  95
   Arg His Gly Thr Tyr Tyr Gly Met Thr Thr Thr Gly Asp Ala Leu Asp
               100                 105                 110
   Tyr Trp Gly Gln Gly Thr Ser Val Thr Val Ser Ser
           115                 120
<210> 10
   <211> 109
   <212> PRT
   <213> Artificial Sequence
<220>
   <221> source
   <223> /note="Description of Artificial Sequence: Synthetic polypeptide"
<400> 10
   Gln Ile Val Leu Thr Gln Ser Pro Ala Ile Met Ser Ala Ser Leu Gly
   1               5                   10                  15
   Glu Arg Val Thr Met Thr Cys Thr Ala Ser Ser Ser Val Ser Ser Asn
   20 25 30
   Tyr Leu His Trp Tyr Gln Gln Lys Pro Gly Ser Ala Pro Asn Leu Trp
   35 40 45
   Ile Tyr Ser Thr Ser Asn Leu Ala Ser Gly Val Pro Ala Arg Phe Ser
   50 55 60
   Gly Ser Gly Ser Gly Thr Ser Tyr Ser Leu Thr Ile Ser Ser Met Glu
   65 70 75 80
   Ala Glu Asp Ala Ala Thr Tyr Tyr Cys His Gln Tyr Leu Arg Ser Pro
   85 90 95
   Pro Thr Phe Gly Gly Gly Thr Lys Leu Glu Ile Lys Arg
   100 105
<210> 11
   <211> 451
   <212> PRT
   <213> Artificial Sequence
<220>
   <221> source
   <223> /note="Description of Artificial Sequence: Synthetic polypeptide"
<400> 11
   Gln Val Gln Leu Lys Glu Ser Gly Pro Gly Leu Val Ala Pro Ser Gln
   1 5 10 15
   Ser Leu Ser Ile Thr Cys Thr Ile Ser Gly Phe Ser Leu Thr Asp Tyr
   20 25 30
   Gly Val His Trp Val Arg Gln Pro Pro Gly Lys Gly Leu Glu Trp Leu
   35 40 45
   Val Val Ile Trp Ser Asp Gly Ser Ser Thr Tyr Asn Ser Ala Leu Lys
   50 55 60
   Ser Arg Met Thr Ile Arg Lys Asp Asn Ser Lys Ser Gln Val Phe Leu
   65 70 75 80
   Ile Met Asn Ser Leu Gln Thr Asp Asp Ser Ala Met Tyr Tyr Cys Ala
   85 90 95
   Arg His Gly Thr Tyr Tyr Gly Met Thr Thr Thr Gly Asp Ala Leu Asp
   100 105 110
   Tyr Trp Gly Gln Gly Thr Ser Val Thr Val Ser Ser Ala Ser Thr Lys
           115                 120                 125
   Gly Pro Ser Val Phe Pro Leu Ala Pro Cys Ser Arg Ser Thr Ser Glu
       130                 135                 140
   Ser Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser Leu Ser Ser Val
               180                 185                 190
   Val Thr Val Pro Ser Ser Ser Leu Gly Thr Lys Thr Tyr Thr Cys Asn
           195                 200                 205
   Val Asp His Lys Pro Ser Asn Thr Lys Val Asp Lys Arg Val Glu Ser
       210                 215                 220
   Lys Tyr Gly Pro Pro Cys Pro Ser Cys Pro Ala Pro Glu Phe Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser Gln
               260                 265                 270
   Glu Asp Pro Glu Val Gln Phe Asn Trp Tyr Val Asp Gly Val Glu Val
           275                 280                 285
   His Asn Ala Lys Thr Lys Pro Arg Glu Glu Gln Phe Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Gly Leu Pro Ser Ser Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Gln Glu Glu Met Thr Lys Asn Gln Val Ser
               355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Arg Leu Thr Val
                   405                 410                 415
   Asp Lys Ser Arg Trp Gln Glu Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Leu Gly Lys
       450
<210> 12
   <211> 215
   <212> PRT
   <213> Artificial Sequence
<220>
   <221> source
   <223> /note="Description of Artificial Sequence: Synthetic polypeptide"
<400> 12
   Gln Ile Val Leu Thr Gln Ser Pro Ala Ile Met Ser Ala Ser Leu Gly
   1               5                   10                  15
   Glu Arg Val Thr Met Thr Cys Thr Ala Ser Ser Ser Val Ser Ser Asn
               20                  25                  30
   Tyr Leu His Trp Tyr Gln Gln Lys Pro Gly Ser Ala Pro Asn Leu Trp
           35                  40                  45
   Ile Tyr Ser Thr Ser Asn Leu Ala Ser Gly Val Pro Ala Arg Phe Ser
       50                  55                  60
   Gly Ser Gly Ser Gly Thr Ser Tyr Ser Leu Thr Ile Ser Ser Met Glu
   65                  70                  75                  80
   Ala Glu Asp Ala Ala Thr Tyr Tyr Cys His Gln Tyr Leu Arg Ser Pro
                   85                  90                  95
   Pro Thr Phe Gly Gly Gly Thr Lys Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 13
   <211> 120
   <212> PRT
   <213> Artificial Sequence
<220>
   <221> source
   <223> /note="Description of Artificial Sequence: Synthetic polypeptide"
<400> 13
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Ala Phe Ser Ser Ser
               20                  25                  30
   Trp Met Asn Trp Val Arg Gln Ala Pro Gly Gln Gly Leu Glu Trp Ile
           35                  40                  45
   Gly Arg Ile Tyr Pro Gly Asp Gly Asp Thr Lys Tyr Asn Gly Lys Phe
       50                  55                  60
   Lys Gly Lys Ala Thr Leu Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Thr Met Ile Ala Thr Gly Ala Met Asp Tyr Trp Gly Gln
               100                 105                 110
   Gly Thr Leu Val Thr Val Ser Ser
           115                 120
<210> 14
   <211> 109
   <212> PRT
   <213> Artificial Sequence
<220>
   <221> source
   <223> /note="Description of Artificial Sequence: Synthetic polypeptide"
<400> 14
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Tyr Thr Gly Val Pro Asp Arg Phe Thr Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Ser Thr Pro Pro
                   85                  90                  95
   Tyr Thr Phe Gly Gly Gly Thr Lys Val Glu Ile Lys Arg
               100                 105
<210> 15
   <211> 450
   <212> PRT
   <213> Artificial Sequence
<220>
   <221> source
   <223> /note="Description of Artificial Sequence: Synthetic polypeptide"
<400> 15
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Ala Phe Ser Ser Ser
               20                  25                  30
   Trp Met Asn Trp Val Arg Gln Ala Pro Gly Gln Gly Leu Glu Trp Ile
           35                  40                  45
   Gly Arg Ile Tyr Pro Gly Asp Gly Asp Thr Lys Tyr Asn Gly Lys Phe
       50                  55                  60
   Lys Gly Lys Ala Thr Leu Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Thr Met Ile Ala Thr Gly Ala Met Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Gln Leu Met Ile
                   245                 250                 255
   Ser Arg Thr Pro Glu Val Thr Cys Val Val Val Asp Val Ser His Glu
                   260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Leu His
               420                 425                 430
   Glu Ala Leu His Asn His Tyr Thr Gln Lys Ser Leu Ser Leu Ser Pro
           435                 440                 445
   Gly Lys
       450
<210> 16
   <211> 215
   <212> PRT
   <213> Artificial Sequence
<220>
   <221> source
   <223> /note="Description of Artificial Sequence: Synthetic polypeptide"
<400> 16
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Tyr Thr Gly Val Pro Asp Arg Phe Thr Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Ser Thr Pro Pro
                   85                  90                  95
   Tyr Thr Phe Gly Gly Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 17
   <211> 119
   <212> PRT
   <213> Artificial Sequence
<220>
   <221> source
   <223> /note="Description of Artificial Sequence: Synthetic
<210> 18
   <211> 112
   <212> PRT
   <213> Artificial Sequence
<220>
   <221> source
   <223> /note="Description of Artificial Sequence: Synthetic polypeptide"
<400> 18
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Val Ser Thr Ser
   20 25 30
   Ser Tyr Ser Tyr Met His Trp Tyr Gln Gln Lys Pro Gly Lys Ala Pro
   35 40 45
   Lys Leu Leu Ile Lys Tyr Ala Ser Asn Leu Glu Ser Gly Val Pro Ser
   50 55 60
   Arg Phe Ser Gly Ser Gly Ser Gly Thr Asp Phe Thr Leu Thr Ile Ser
   65 70 75 80
   Ser Leu Gln Pro Glu Asp Phe Ala Thr Tyr Tyr Cys Gln His Ser Trp
   85 90 95
   Glu Ile Pro Tyr Thr Phe Gly Gly Gly Thr Lys Val Glu Ile Lys Arg
   100 105 110
<210> 19
   <211> 449
   <212> PRT
   <213> Artificial Sequence
<220>
   <221> source
   <223> /note="Description of Artificial Sequence: Synthetic polypeptide"
<400> 19
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Ser Trp Val Arg Gln Ala Pro Gly Lys Gly Leu Glu Trp Val
           35                  40                  45
   Ala Glu Ile Arg Leu Lys Ser Asp Asn Tyr Ala Thr His Tyr Ala Glu
       50                  55                  60
   Ser Val Lys Gly Arg Phe Thr Ile Ser Arg Asp Asp Ser Lys Asn Ser
   65                  70                  75                  80
   Leu Tyr Leu Gln Met Asn Ser Leu Arg Ala Glu Asp Thr Ala Val Tyr
                   85                  90                  95
   Tyr Cys Thr Gly Tyr Tyr Ala Asp Ala Met Asp Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Ile Cys Asn Val Asn His Lys Pro
   195 200 205
   Ser Asn Thr Lys Val Asp Lys Lys Val Glu Pro Lys Ser Cys Asp Lys
   210 215 220
   Thr His Thr Cys Pro Pro Cys Pro Ala Pro Glu Leu Leu Gly Gly Pro
   225 230 235 240
   Ser Val Phe Leu Phe Pro Pro Lys Pro Lys Asp Thr Leu Met Ile Ser
   245 250 255
   Arg Thr Pro Glu Val Thr Cys Val Val Val Asp Val Ser His Glu Asp
   260 265 270
   Pro Glu Val Lys Phe Asn Trp Tyr Val Asp Gly Val Glu Val His Asn
   275 280 285
   Ala Lys Thr Lys Pro Arg Glu Glu Gln Tyr Asn Ser Thr Tyr Arg Val
   290 295 300
   Val Ser Val Leu Thr Val Leu His Gln Asp Trp Leu Asn Gly Lys Glu
   305 310 315 320
   Tyr Lys Cys Lys Val Ser Asn Lys Ala Leu Pro Ala Pro Ile Glu Lys
   325 330 335
   Thr Ile Ser Lys Ala Lys Gly Gln Pro Arg Glu Pro Gln Val Tyr Thr
   340 345 350
   Leu Pro Pro Ser Arg Glu Glu Met Thr Lys Asn Gln Val Ser Leu Thr
   355 360 365
   Cys Leu Val Lys Gly Phe Tyr Pro Ser Asp Ile Ala Val Glu Trp Glu
   370 375 380
   Ser Asn Gly Gln Pro Glu Asn Asn Tyr Lys Thr Thr Pro Pro Val Leu
   385 390 395 400
   Asp Ser Asp Gly Ser Phe Phe Leu Tyr Ser Lys Leu Thr Val Asp Lys
   405 410 415
   Ser Arg Trp Gln Gln Gly Asn Val Phe Ser Cys Ser Val Met His Glu
               420                 425                 430
   Ala Leu His Asn His Tyr Thr Gln Lys Ser Leu Ser Leu Ser Pro Gly
           435                 440                 445
   Lys
<210> 20
   <211> 218
   <212> PRT
   <213> Artificial Sequence
<220>
   <221> source
   <223> /note="Description of Artificial Sequence: Synthetic polypeptide"
<400> 20
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Val Ser Thr Ser
               20                  25                  30
   Ser Tyr Ser Tyr Met His Trp Tyr Gln Gln Lys Pro Gly Lys Ala Pro
           35                  40                  45
   Lys Leu Leu Ile Lys Tyr Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln His Ser Trp
                   85                  90                  95
   Glu Ile Pro Tyr Thr Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 21
   <211> 15
   <212> PRT
   <213> Artificial Sequence
<220>
   <221> source
   <223> /note="Description of Artificial Sequence: Synthetic peptide"
<400> 21
   Gly Gly Gly Gly Ser Gly Gly Gly Gly Ser Gly Gly Gly Gly Ser
   1               5                   10                  15